(19)

**Europäisches Patentamt**
**European Patent Office**
**Office européen des brevets**

(11)  **EP 2 864 048 B1**

(12)  **EUROPEAN PATENT SPECIFICATION**

(45) Date of publication and mention
of the grant of the patent:
**09.11.2016  Bulletin 2016/45**

(51) Int Cl.:
***B01L 3/02*** *(2006.01)*        ***B01L 3/00*** *(2006.01)*

(21) Application number: **13733418.1**

(22) Date of filing: **25.06.2013**

(86) International application number:
**PCT/GB2013/051668**

(87) International publication number:
**WO 2014/001781 (03.01.2014 Gazette 2014/01)**

(54) **MICROFLUIDIC DEVICE FOR DROPLET GENERATION**

MIKROFLUIDISCHES SYSTEM ZUR ERZEUGUNG VON TRÖPFCHEN

SYSTEME MICROFLUIDIQUE POUR CRÉATION DE GOUTTELETTES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.06.2012  GB 201211342**

(43) Date of publication of application:
**29.04.2015  Bulletin 2015/18**

(73) Proprietors:
• **Imperial Innovations Limited
London, Greater London SW7 2PG (GB)**
• **Cambridge Enterprise Ltd.
Cambridge
Cambridgeshire CB2 1TN (GB)**

(72) Inventors:
• **GIELEN, Fabrice Matthieu
Cambridge
Cambridgeshire, CB2 1GA (GB)**
• **VAN VLIET, Liisa Darnell
Cambridge
Cambridgeshire, CB2 1GA (GB)**

• **EDEL, Joshua Benno
London
Greater London, SW7 2AZ (GB)**
• **DEMELLO, Andrew James
8093 Zürich (CH)**
• **NIU, Xize
Southhampton
SO17 1BJ (GB)**
• **DEVENISH, Sean Richard Anthony
Cambridge
Cambridgeshire, CB2 1GA (GB)**
• **HOLLFELDER, Florian
Cambridge
Cambridgeshire, CB2 1GA (GB)**

(74) Representative: **Coles, Andrea Birgit et al
Kilburn & Strode LLP
20 Red Lion Street
London WC1R 4PJ (GB)**

(56) References cited:
**US-A1- 2009 288 710     US-A1- 2010 173 394
US-A1- 2012 039 774**

## Description

TECHNICAL FIELD

This invention relates to a microfluidic system for generating droplets and allows merging of droplets in a controlled and synchronised manner, in order to achieve an improved analytical device and methods.

BACKGROUND ART

There has been a continued desire for using smaller sample quantities, which has led to research being carried out into transferring many known analytical techniques to the sub-millimetre scale. Recent advances in microfluidic control architecture have significantly improved the ability to work with materials on a microscale, for example, allowing precise temporal and spatial manipulation of single droplets and functions such as sorting, splitting and merging droplets for complex analyses.

Droplet-based microfluidics has emerged as a valuable platform for performing high throughput chemical and biological experiments. In such systems, droplets are made to spontaneously form when laminar streams of aqueous reagents are injected into an immiscible carrier fluid, either at a T-junction or in a flow focusing geometry.

Biological processes in microdroplets have been described in Current Opinion in Chemical Biology, 2010, 14:1-8; Mol. BioSyst, 2009, 5, 1392-1404; and Lab Chip, 2008, 8, 1244-1254. Processes that have been carried out inside droplets include DNA amplification, protein expression, enzymatic turnover, cell growth and multicellular organism growth.

PRIOR ART

Known microfluidic devices are associated with various practical difficulties, including low versatility, high cost and sample cross-contamination.

US 2009/0288710 discloses a microfluidic sampling system for taking samples of fluid from at least 2 wells from above, using a microsampling head. A first well, contains, e.g., a sample covered by a second fluid (e.g. an oil) that is immiscible with the first fluid. A second well contains, e.g., a reagent, covered by a second fluid (e.g. an oil). The microsampling head draws in a volume of the sample from the first well, a volume of the second fluid of the first well, and is then moved to the second well to draw in a volume of the reagent of the second well. A third fluid, acting as a wash fluid, may cover both wells such that they are in fluid communication. This system addresses cross contamination by virtue of the wash fluid, evaporation of the sample by virtue of the second fluid layer over the first fluid layer, and unwanted introduction of air by virtue of keeping the wells in fluid communication by means of the third fluid.

Series of alternating droplets are prepared by moving the microsampling head between different wells to draw in different samples interspersed by carrier fluid. Pairs of consecutive droplets (e.g. sample and reagent), separated from each other by the second fluid in the microsampling channel, may be combined by virtue of a downstream increase in the cross section of the microsampling channel, which is large enough so that the droplet of the second fluid is no longer able to fully separate the two consecutive droplets, which then mix.

A valve system is used to achieve droplet sampling and to conduct the droplets into the system. The valve is set to a first position, to pull a droplet out of the sampling reservoir. When the droplet passes the valve, the valve is switched, to push the droplet onwards through the valve and along the tubing into the downstream system.

There are various practical difficulties associated with this device, including contamination around the valve joint, the need to use large sampling volumes, and the slowness of the system, with a relatively low throughput. Data in US 2009/0288710 shows that samples are taken only once every 60 seconds. This indicates the large spacing present between sample droplets in the system.

Droplet dilution has been reported using a passive geometry in which a trapped mother droplets is diluted down by a series a dilution droplets. Other dilution mechanisms include the creation of a chemical gradient using diffusion and laminar flows before encapsulation in an oil phase. (Ref: Niu et al., Nature Chemistry (2011), 3; 437-442)

US 7,129,091 discloses a microfluidic device for manipulating fluid plugs in channels. Both the merging of a plurality of fluid channels into a single fluid channel, and the splitting of fluid plugs into two or more channels is disclosed. Mixing of fluids by generating chaotic or turbulent flow by channel geometry is also disclosed.

'Plugs' of fluids containing different reagents can be merged by passing them through a T-shaped region in a channel and having the droplets meet head on or travel at different velocities. Pairs of plugs can be merged by controlling the arrival time of plugs flowing in opposite direction towards a common area, so that the plugs arrive 'at around the same time' to form a single plug. The sequential arrival of droplets into a merging chamber is also disclosed. The exact arrival time, and exact order of droplet arrival is said to be difficult to control.

US 2006/0094119 discloses a system for microfluidic manipulation of small volumes of solution. The system is configured into a loading component, a holding component, a combining component and a receiving component.

The detachable holding component may hold the droplets in a linear array. Two arrays of droplets can be stored such that they may be merged, and the merged plugs transferred to the combining component for further reactions. Each holding component includes a series of different reagents. The combining component can be used to merge plugs with other plugs, such as a stream of reagent plugs, for example, by injection.

The droplets may be transferred to a receiving component, such as a microchannel, e.g., a length of tubing. Reactions in plugs can be monitored in any of the components of the system or after exiting the system.

In the prior art systems it is very difficult to accurately control the merging of droplets. The present invention represents a significant improvement over the current state of the art in providing a simple device, able to generate droplets from very small sample volumes in a controlled manner, merge droplets in a controlled manner, generate large amounts of very accurate data for chemical and biological studies, and operate at a low cost compared to the current standard devices.

SUMMARY OF THE INVENTION

The present invention relates to a microfluidic system for the controlled generation of droplets of a sample fluid as claimed in claim 1. The system comprises:

- at least one reservoir containing a sample fluid and a carrier fluid;
- one or more droplet generators, each comprising an opening, or 'microsampling tip', for the controlled sampling of droplets from a sample reservoir; and
- tubing or capillary to conduct the droplets in the carrier fluid; and
- a source of pressure.

Optionally, the invention may further comprise a microfluidic chip supporting channels or capillaries to conduct one or more droplets in a carrier fluid. The tubing or capillary may connect the microsampling tip with the microfluidic chip.

Microfluidic chips suitable for the current invention are known in the art. They may be made of glass or polymer and fabricated using standard microfabrication techniques adapted from the semiconductor industry. Soft lithography in particular has been extensively used by the microfluidics community. See "Soft Lithography in Biology and Biochemistry", Whitesides, G.M., Ostuni, E.S., Takayama, S., Jiang, X., and Ingber, D.E., Ann. Rev. Biomed. Eng, 2001, 3, 335-373. Suitable examples include glass or plastic chips from Dolomite Microfluidics, ChipShop, Micronit, Micralyne, ThinXXS, Ibidi. These chips include a variety of functions in either 2-phase or continuous flow.

The present invention relates to a droplet generator.

The device comprises an electronically driven microsampling head, which comprises an opening, or 'microsampling tip', for generating droplets of fluid. The opening (microsampling tip) is positioned under a container, reservoir or tank, loaded with at least two fluid phases, wherein adjacent phases are immiscible with each other. The microsampling tip is moveable relative to the container, reservoir or tank. The microsampling tip is optionally movable at least laterally and vertically. This can be achieved by electronic control and may be preprogrammed.

Fluid aspirated by, or pushed through, the microsampling tip passes to a microfluidic chip, via a section of tubing or a capillary connecting the microsampling tip with the microfluidic chip. Optionally, a connector means may be incorporated between the tubing or capillary and the microfluidic chip.

Pressure is applied to the system in order to achieve aspiration of droplets by the microsampling tip into the system. In one embodiment, negative pressure is applied to the system at the far end of the tubing or capillary system on the side of the microfluidic chip distant from the tip. When the microsampling tip is positioned in a fluid phase, the application of negative pressure causes fluid to be aspirated into the tip and flow into the tubing. In another embodiment, positive pressure is applied. In a further embodiment, positive and negative pressure are both applied to the system.

By moving the microsampling tip between a first fluid phase and a second fluid phase, and back into the first phase, droplets are aspirated and pulled into the tubing. Repeated moving of the tip between the first and second fluid phases aspirates a series of droplets of a first fluid phase, with each pair of droplets separated by a volume of the second fluid phase. The retention time of the microsampling tip in each phase, in conjunction with the size and shape of the tubing or capillary, determines the size of droplets generated and conducted into the tubing.

The system of the invention may be configured to be used as a droplet synchroniser.

In this embodiment, the droplet generator comprises at least two electronically driven openings ('microsampling tips'), which aspirate droplets from at least one sample well. The number of openings (microsampling tips) corresponds to the number of samples. Each microsampling tip in the droplet synchroniser system is dedicated to aspirating droplets from only one type of sample. A tubing section is associated with each microsampling tip to allow parallel droplet generation. The generation of droplets occurs in the same manner as described above for the droplet generator system.

Droplets can be merged with each other on the microfluidic chip or in a capillary in a controlled fashion. Various merging methods are possible and envisaged by the present invention. For example, on-chip or in-capillary merging allows for controlled merging of two or more sequential droplets, and also allows for one or more droplets to be merged with one or more droplets generated in parallel by different microsampling tips. Further, it is also possible to control the merging of a droplet from a first sample well and a droplet from a second sample well into one combined droplet.

Pairs, triplets, quadruplets etc. of droplets are generated in sequence in a capillary using the described robotic head. The relative distances between droplets will depend on capillary geometry, droplet size, speed, viscosity and interfacial tension between the oil and droplet. By tuning these parameters, merging can be obtained in a controlled manner and get the pairs, triplets, quadruplets etc. of droplets to merge after travelling a desired distance within the capillary.

Droplets can be delivered into a capillary and merged in a controlled fashion. For example, a first droplet can be generated from a first sample, and a second droplet can be generated from a second sample - both generated at low flow rate. The droplet can merge with any of the available sample fluid, including the first or second sample, again to generate a third, fourth or fifth droplet. The droplets can then be merged into one or more combined droplets in a controlled fashion directly in capillary. This merging can, for example, be achieved by subsequently increasing the flow rate. Combinatorial mixing and dilution gradient can thus be achieved.

Droplets can be delivered to one location or multiple locations on the microfluidic chip, in a controlled fashion. For example, droplets delivered to the microfluidic chip from a first tubing, associated with a first sampling fluid, can be delivered to a channel on the microfluidic chip at a location that is the same as, or different to, the location at which droplets from a second sampling fluid (and/or any subsequent sampling fluids) are delivered to a channel on the microfluidic chip.

Advantages of the system of the invention include:

- droplet size can be accurately pre-determined
- droplets can be generated at high speed (for example, 1 Hz - 1 kHz, for example, 1 Hz - 100Hz)
- no contamination of droplets is observed, even at high sampling speeds
- only very small sample volumes are required to generate large numbers of sample droplets (for example 5 - 40 $\mu$L sample volume)
- droplets of different sizes can be created (for example picolitre - 100 nanolitre range), and droplet size can be controlled for each individual droplet
- spacing between the droplets can be accurately controlled
- droplet generation can be synchronised, alternated or swept
- droplets generated by different microsampling tips can be delivered to one location or multiple locations of the microfluidic chip in a controlled, 'synchronised' manner, thus allowing for controlled droplet merging
- the ability to carry out on-demand droplet generation, delivery and merging.

In the description below, the term "microdroplet" or "droplet" incorporates droplets of micro-, nano-, pico-, femto-, and attolitre volume.

BRIEF DESCRIPTION OF THE FIGURES

Figures 1 B and C show a sampling well and microsampling head, with sampling occurring from below.

Figure 2 shows multiple sample containers held by a carousel, which corresponds to the arrangement of Figure 1C.

Figure 3 shows an overview of sampling and droplet generation from two different sample containers, using the carousel of Figure 2.

Figure 4 shows an overall set up of the system of the invention.

Figure 5 shows the droplet generator / sampling robot configuration.

Figure 6 shows the droplet synchroniser configuration.

Figure 7 shows a set-up of the droplet synchroniser

Figure 8 shows an expanded view of droplet merging.

Figure 9 shows a series of different droplets separated by carrier fluid, in a tubing or capillary.

Figures 10-14 show the system configuration for experimental examples.

Figures 15-19 relate to worked examples of the invention.

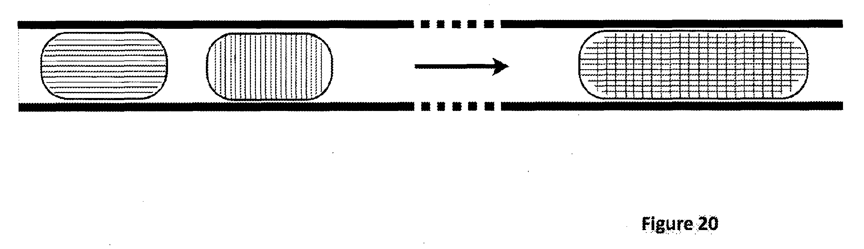

Figure 20 shows in-capillary merging of two, three, or more droplets by increased flow rate.

DETAILED DESCRIPTION

The system of the present invention provides for droplet generation, dilution (on-chip or in capillary), merging (on-chip or in capillary), and separation of droplets (on-chip or in capillary). The system comprises:

- a microfluidic chip supporting channels or capillaries to conduct one or more droplets in a carrier fluid;
- one or more droplet generators, each comprising an opening ('microsampling tip') as part of a microsampling head, for the controlled sampling of droplets from a sample reservoir;
- one or more sections of tubing or capillary, optionally connecting the microsampling tip(s) with a microfluidic chip, and for conducting the droplets from the microsampling tip to the microfluidic chip; and

- a source of pressure, that is, negative and/or positive pressure.

The system of the present invention comprises a droplet generator, optionally connected to a microfluidic chip via a section of tubing or a capillary. In the description below, the term 'tubing' is used to refer to either tubing or a capillary. The droplet generator comprises an electronically driven microsampling head, comprising an opening ('microsampling tip'), for aspirating droplets of fluid. The microsampling tip is movable relative to the container, well, reservoir or tank, and optionally movable at least laterally and vertically. The droplet generator opening (microsampling tip) is positioned under a container, well, reservoir or tank, loaded with at least two fluid phases that are immiscible with each other. In one embodiment the droplet generator opening (microsampling tip) is positioned under a container, well, reservoir or tank, that is, the drawing of a sample occurs from below.

When used in a droplet synchronising configuration, the droplet generator comprises at least two electronically driven microsampling tips (openings). The total number of tips corresponds to the number of different fluid sample types being sampled. The tips are driven by an electronic means, such as a motor or an electromagnetic actuator, and are controllable independently of each other. Alternatively, the sample containers are moveable with respect to the microsampling tips.

When the system is configured to enable synchronised sampling and merging of droplets, the number of microsampling tips, affixed to a dedicated section tubing or capillary, corresponds to the number of samples, and each such robotic sampling 'arm' is independently controllable.

The tip (opening) may be in the shape of a hollow cylindrical tube, having a first and a second end. The first end of the tip is open and is dipped into a fluid sample contained in a sampling well. A section of tubing is either fixed to the second end of the tip, or the tip may consist of the tubing. The shape of the tip is preferably circular, with either a circular flat or asymmetric opening.

When the tip, or open end of the tubing, is positioned in a fluid phase, the application of pressure (for example, negative and/or positive pressure) causes a volume of the fluid to be aspirated through the tip and into the tubing. Negative pressure is typically applied at a location distant from the tip and positive pressure is typically applied at a location close to the tip.

The system may be configured such that the microsampling tip aspirates fluid samples from a container from below. In one embodiment, the sample-filled container is movable with respect to the microsampling tip, and the microsampling tip is fixed in position. In a second embodiment, the microsampling tip is moveable with respect to the sample-filled container.

By moving the first end of the microsampling tip within a sample-filled container from a first fluid phase into a second fluid phase, and back into the first fluid phase, under application of constant negative pressure, a sample droplet will be aspirated and pulled into the tubing.

The first phase may, for example, be a carrier fluid, and the second phase may be a sample fluids. Repeatedly moving the microsampling tip between the first and second phases creates droplets of sample fluid, interspersed by carrier fluid. The size and spacing of droplets may be determined by the length of time the tip is resident in each phase.

The carrier fluid may, for example, be an oil, immiscible with an aqueous sample fluid, or vice versa. The surface of the sampling component is ideally hydrophobic.

The term 'sample' as used herein means a sample of a reagent, compound, substrate, enzyme, cells, or any other component to be analysed.

Reaction volumes are in the pico- to nanolitre range ($10^{-12}$ to $10^{-9}$L). Droplets may be in the range of 1-100 nL, for example, 10-70 nL, for example, 20-50 nL.

The dead volume (i.e. lost sample) in the present invention is zero, because sample may be re-used.

In a further aspect, when the system is configured for droplet synchronisation, the droplet generator comprises at least two, for example, at least three, or at least four independently controllable arms, each comprising an electronically driven microsampling tip, and a section of tubing for conducting the droplets to the microfluidic chip. The microsampling tips may all be provided on the same microsampling head, or may each be provided on separate microsampling heads.

In a further embodiment, when the system is configured for droplet synchronisation, the droplet generator comprises at least two, for example, at least three, at least four or at least five, independently controllable arms, corresponding to the number of sample wells or sample types provided.

Preferably, the droplet synchronising system comprises two or three independently controllable arms.

Droplets of each sample type are generated into separate tubing attached to each respective tip. The tubing is connected to a receiving component. The receiving component may, for example, be an extension of tubing, a microfluidic chip, one or more collection vials, a syringe pump, or an analytical instrument. The tubing may also be connected directly to a source of negative pressure (such as a syringe pump) and/or a system outlet.

In one embodiment, the droplet generator will aspirate droplets taken from different samples. Contamination is minimised due to the tip passing through the oil phase, and due to the material of the tip.

In another embodiment, when the system is configured for droplet synchronisation, each tip of the droplet generator is dedicated to use with only one sample, or one type of sample, in order to allow for high speed sampling. For example, a first tip is used for sampling droplets of substrate from a sampling well, or wells, containing a substrate, a second tip is used for sampling droplets of a reagent from a sampling well, or wells, containing a reagent, a third tip is used for sampling droplets of buffer from a sampling well, or wells, containing a buffer.

The samples may be provided in containers carried by a carousel holding a plurality of sample containers, such as Eppendorf tubes. The system of the present invention can also be set up to sample droplets from microtitre plates, such as a 96-well plate, a 384-well plate, or a 1536-well plate. Each sample container, or well, may contain a different sample, for example, each well may contain a different compound from a chemical library. The term 'container' will be used to refer to both sample containers and wells in this description.

In use, either a carousel holding the sample containers is rotated (or the well plate is moved) with respect to a microsampling tip, or a microsampling tip can be moved with respect to sample containers.

The microsampling tip of the droplet generator sequentially aspirates droplets from each sample container. The device can be programmed to aspirate one droplet or a plurality of droplets from each sample container. The device may be programmed to aspirate a predetermined different number of droplets from different sample containers.

Droplets from multiple sample containers may be aspirated into the tubing. As an example, one droplet from each of a series of different sample containers may be aspirated, so that a series of droplets, each from a different sample, is aspirated into the tubing. Alternatively, a plurality of droplets (a series) of a first sample A, may be followed by a series of droplets of a second sample B, etc. Each pair of sample droplets is interspersed by a volume of carrier fluid.

A microsampling head and tip, and the tubing (or capillary) attached thereto, which connects the microsampling tip to the microchip, makes up a robotic 'arm', which is controlled by a microcontroller. The movement of each arm is independently programmable via the microcontroller which controls retention time in each fluid phase. The whole system has a CPU controlling the pressure and which can feedback to the microcontroller of the robotic arms.

Each section of tubing conducts droplets from the microsampling tip to a microchannel (or capillary) on a microfluidic chip. Separate microchannels on the microfluidic chip converge into one microchannel via convergence points. Alternatively, experiments can be run with droplets which are not merged with another droplet. For example, a reaction may be started just prior to loading the droplets into the system. The reaction progress is then simply monitored in each generated droplet of the reaction mixture.

The present invention enables droplets from a robotic arm to be delivered to a convergence point in a synchronised manner, rather than in a simple flow-dependent sequential manner. The synchronised delivery of 1 to N droplets to a convergence point provides a 'one-step' coalescence format based on pressure equilibrium in each of the microchannels.

This synchronised arrival time of droplets from the microchannels at a convergence point enables two or more droplets to merge into one 'mixed droplet'. A reaction may be initiated in each mixed droplet upon merging, by the sample and the reagent coming into contact with each other. This can also occur directly in capillary. Each synchronised arrival of one droplet from each of the microchannels at the convergence point leads to the creation of one mixed droplet. Sequential mixed droplets in the series are completely separated from each other by a volume of the oil (carrier fluid).

This synchronisation allows droplets to arrive at a convergence point at a predetermined time. Thus, not only can two or more droplets be controlled to arrive at the convergence point at a substantially identical time, but the time of arrival of each droplet may also be accurately controlled, allowing a first and second droplet to arrive at a convergence point a predetermined length of time apart. Optionally three or more droplets can be merged by arriving at a convergence point at the same time.

Each (optionally mixed) droplet proceeds along a channel on the microfluidic chip to a receiving component where the progress of a reaction can be monitored. Alternatively, each optionally mixed droplet is monitored in capillary. Alternatively, the droplet may proceed along the channel to a receiving component for storage. The receiving component may be a length of tubing. This tubing may be connected at one end to the outlet of the system or to a channel on a microfluidic chip, or it may be a microchannel or microstructure on a microfluidic chip. Droplets (optionally mixed) may alternatively be deposited on a surface, e.g. a MALDI plate, a bead plate for emulsion PCR, or be sprayed, e.g. in electrospray ionisation.

In the prior art, sequential arrival of droplets in a microchannel suffers from the necessity of drawing sample and reagent alternately, and thus necessitates the inclusion of a wash step to minimise or avoid cross-contamination of the different samples. The sequential arrival systems of the prior art also suffer from time delay in between the droplets, which limits the throughput of the system.

With the present invention, high sample throughput is possible. The invention allows for creating a sequence of identical droplets (e.g. reagent or sample) in one capillary for subsequent merging with a series of non-identical droplets (e.g. sample or reagent) and, also for merging these droplets with a series of droplets of a further component, such as a further reagent, or a buffer. These droplets may be identical or non-identical, and may be of the same or a different volume to each other.

Independent control over each of the robotic arms of the microfluidic system is possible, to control frequency of sampling, droplet volume, droplet spacing, number of capillaries being used, speed of droplet flow etc.

Merging of droplets can be pre-programmed for timed reactions to occur. For example, a first droplet (A) and second droplet (B) can be merged first, before the resulting mixed droplet (A+B) is merged with a third droplet (C). Alternatively, droplets A, B and C can be controlled to arrive at a convergence point at precisely pre-determined time-spacings (in-capillary or on-chip) to merge droplets A+B+C together at the same time. By controlling the movement of the droplets in this precise way, no droplet of sample will miss receiving a volume of reagent (and/or buffer), thereby achieving reliability of experimental results.

Merging sequences of droplets can be programmed for timed reactions. Further droplet merging may be carried out. Size, material and design of the tubing or microfluidic channels are important to achieving this function.

DESCRIPTION WITH REFERENCE TO THE FIGURES

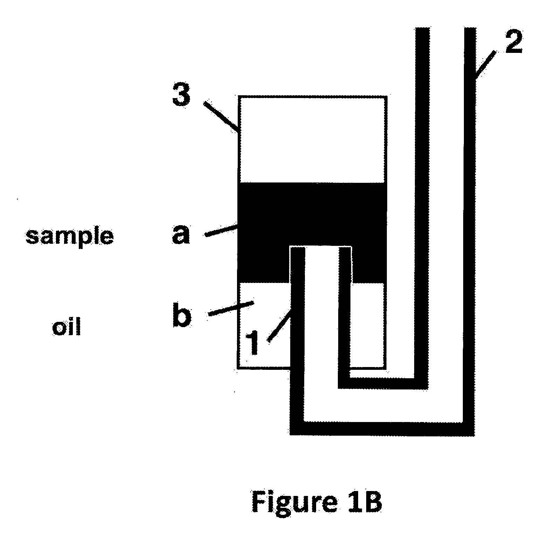

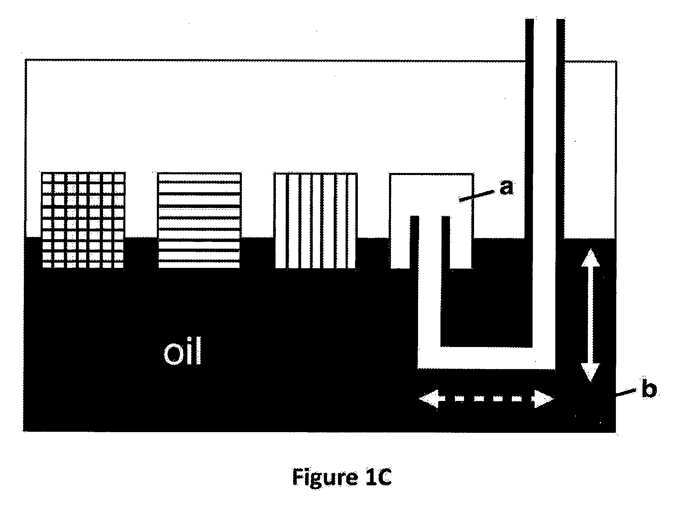

Droplet generation can be achieved from below as shown in Figure 1B and C.

In Figure 1B, the microsampling tip (1) enters the sampling chamber (3) from below, and repeatedly moves vertically between fluid (a) (4) and fluid (b) (5), which are immiscible with each other. The moving of the microsampling tip between fluid (a) and fluid (b), and the constant application of pressure to the system, results in volumes of fluid (a) and fluid (b) being alternately aspirated into the microsampling tip (1) and flowing into the tubing (2), thereby creating a series of droplets of fluid (a) separated by fluid (b) in the tubing. In an alternative embodiment, the microsampling tip is stationary and the sample container is moved, to move the microsampling tip between fluid (a) and fluid (b).

In a further alternative embodiment, the base of the sampling chamber shown in Figure 1C has a small opening and is placed in a larger reservoir containing fluid (b). The sampling chamber containing fluid (a) is sealed at the top, to avoid sample evaporation, with only a small hole positioned above the surface level of fluid inside and surrounding the sampling chamber, to allow for pressure equilibration within the sampling chamber, as fluid (a) is removed during sampling. In this embodiment, when multiple sampling chambers are held by a carrousel or in a microtitre plate, the bases of all the sampling chambers may be placed in a general reservoir of fluid (b), as shown.

In one aspect, fluid (b) is an oil and fluid (a) is a sample. In a further embodiment, if the container is open above the sample, sample fluid (a) may be covered with an oil layer, with an oil with a density lower than the sample, to prevent evaporation of the sample.

The provision of an oil layer on top of and/or beneath the sample fluid avoids the need for a washing fluid. Tests carried out by the Applicant have shown that there is no cross-contamination between droplets sampled by the device of the present invention.

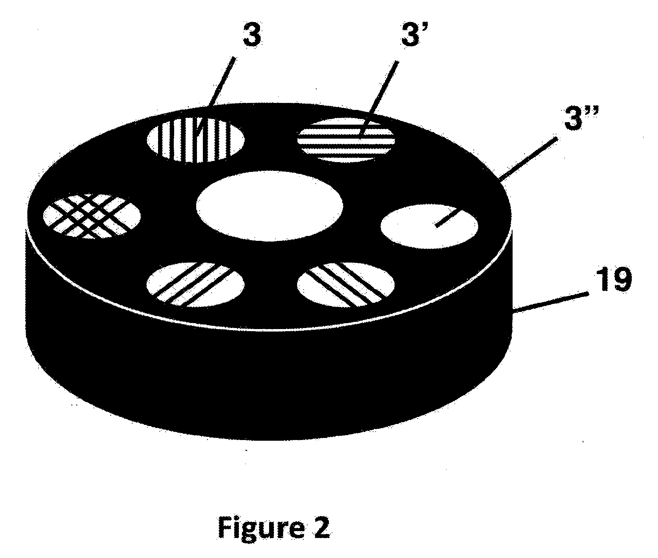

**Figure 2** shows multiple sample containers (3, 3', 3") held by a carrousel (19), which corresponds to the arrangement of Figure 1C.

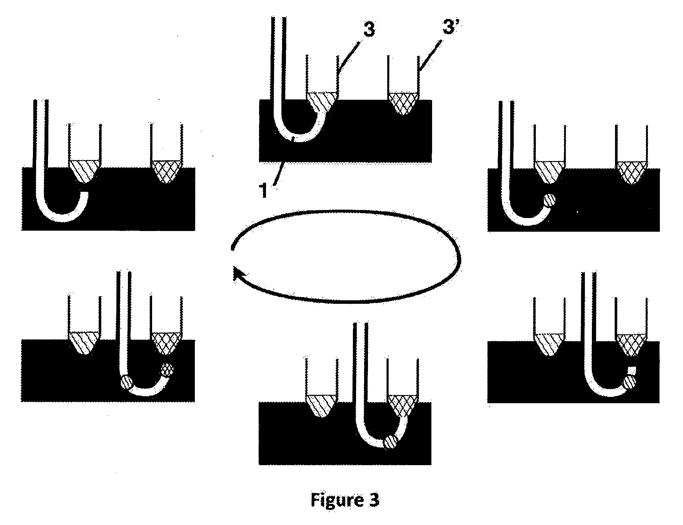

**Figure 3** shows a step-by-step overview of sampling and droplet generation from two different sample containers (3, 3'), using for example the carousel of Figure 2. This can be adapted to a multi-well format (e.g. 96; 384, 1536- wells)

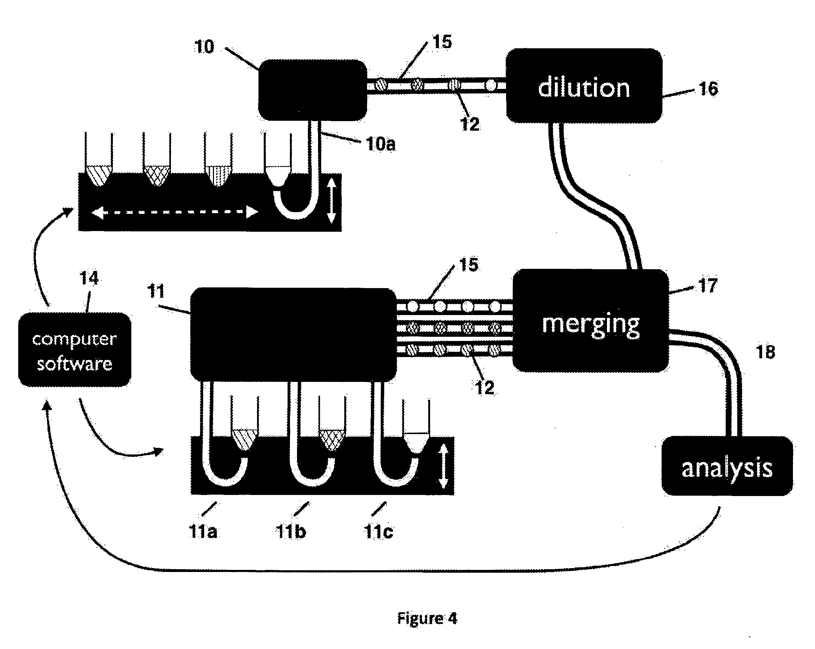

**Figure 4** shows an overview of a system of the present invention. The system can be configured to comprise a droplet generator (10), or as a droplet synchroniser (11). The robotic sampling arms of the droplet generator (10a), or droplet synchroniser (11 a, b, c) are each independently controlled by a microcontroller (computer) (14). Sampled droplets (12) are aspirated into the tubing (15) and flow towards a microfluidic chip (16, 17), where droplets are diluted or merged. Merged droplets flow into a receptor (18), such as tubing, where they may be analysed. Analysis of the droplets may be carried out in the tubing, for example, by laser analysis, absorbance measurements or fluorescence imaging.

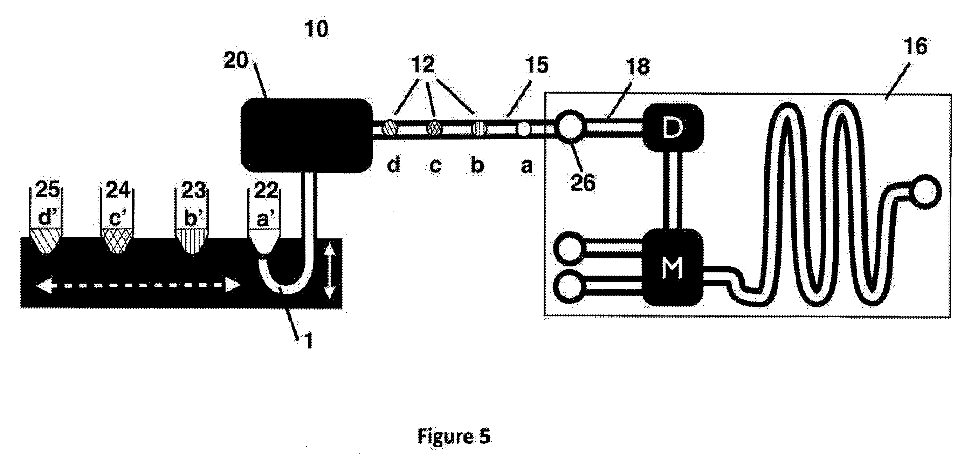

**Figure 5** shows the microsampling head (20) of the droplet generator configuration (10) of Figure 4. The microsampling tip (1) aspirates sample droplets (12) from a sequence of sample wells (22-25). Either the microsampling head or the sample wells, or both, are movable, i.e. they are moveable with respect to each other. The droplets (a, b, c, d) are aspirated sequentially in a desired order from the sample wells (22-25; a', b', c', d') into tubing (15). The tubing is joined to a microchannel (18) on a microfluidic chip (16) by a connecting means (26), to allow the droplets to continue to flow along the microchannel.

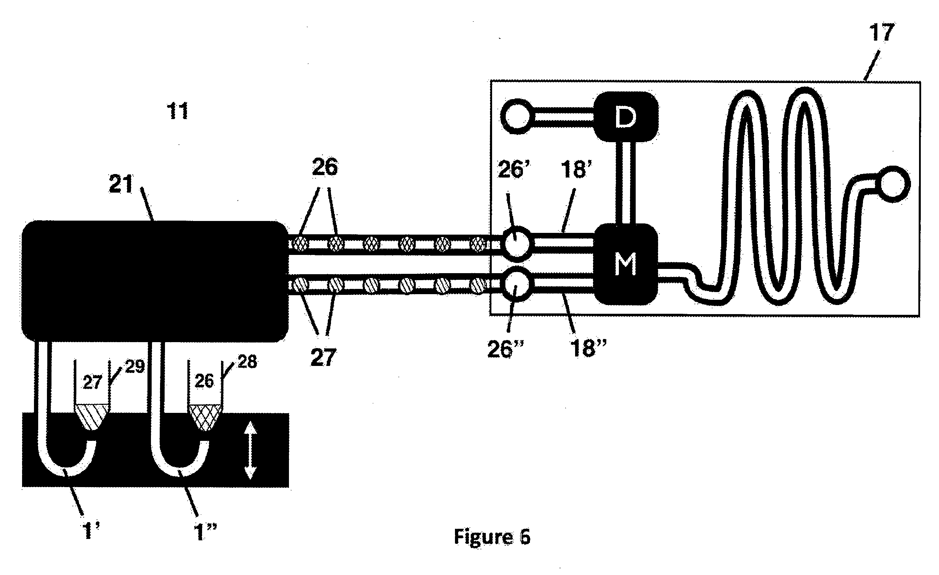

**Figure 6** shows a microsampling head (21) of the droplet synchroniser configuration (11) of Figure 4. The microsampling tips (1', 1") aspirate sample droplets (26, 27) from two respective sample wells (28, 29). The droplets (26, 27) are aspirated from the sample wells (28, 29) into tubing (15). The tubing is joined to microchannels (18', 18') on a microfluidic chip (17). Microchannels conducting droplets from different robotic arms are joined at one or more convergence points (26', 26").

The systems described in Figures 5 and 6 may be combined on a single microchip, allowing merging of sample droplets from samples 22-25 to merge with droplets of samples 26 and 27, as shown.

Approaches for droplet merging use either passive, active or passive/active mechanisms. Passive droplet trapping relies on the use of geometrical constraints to bring two or more droplets into contact. These include *inter alia* channel enlargment, use of pillar arrays, use of hydrophilic patches, surface tension, viscosity, size can also be used to achieve merging. Active merging mechanisms include *inter alia* electric fields (electrocoalescence), magnetic fields, acoustic waves, pneumatic valves. Hybrid droplet merging, combining pillars and electric fields, has been reported in the literature.

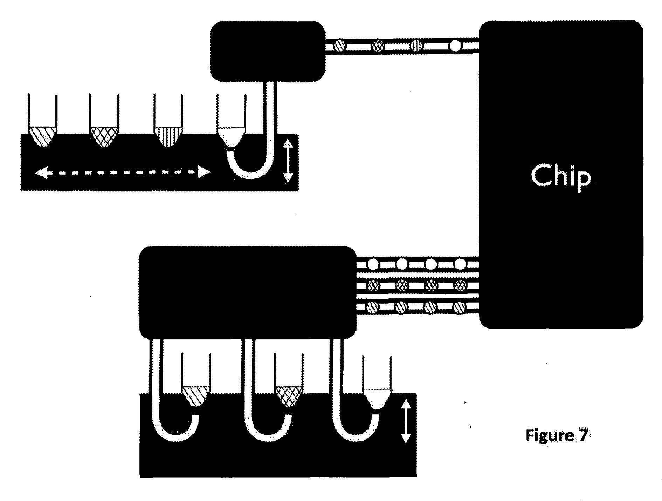

**Figure 7** shows a system set up to i) generate droplets of a series of different samples, such as different compounds of a chemical library; and ii) generate a first, second and third series of droplets of three different sample types. These series of droplets may, for example, be a buffer, an enzyme, and a substrate, respectively. The droplets are conducted to channels of a microfluidic chip, via tubing. On the microfluidic chip, channels are laid out to allow for merging of droplets. The system of the present invention allows for this merging to be carried out in a precise, timed, pre-determined manner.

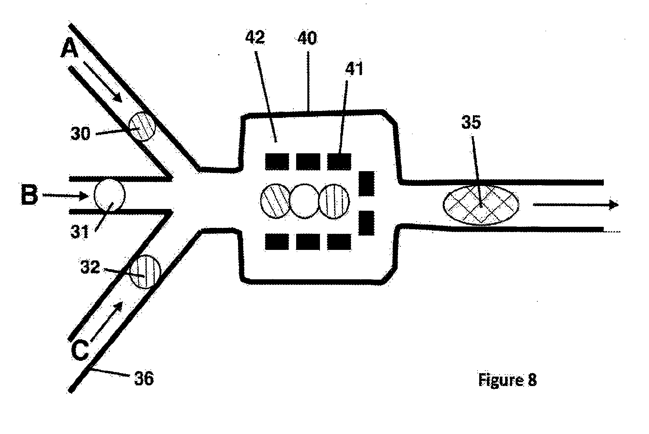

**Figure 8** shows a passive method for merging three droplets into one droplet in a synchronised manner. In an aspect of the invention, droplets can be delivered to one location or multiple locations on the microfluidic chip, in a controlled fashion. The microfluidic chip may be provided with one or more connecting means, to allow the droplets to flow from the tubing, into a channel on the microfluidic chip.

Referring to Figure 8, droplets A (30), B (31) and C (32) can be controlled to arrive at a convergence point at the same time, in order to merge into one droplet (35). Alternatively, a droplet (C) flowing along a channel (36) provided on the microfluidic chip, may be first merged with a droplet of a first sample (A), continue to flow along the channel, and subsequently be merged with a droplet of a second sample (B). Droplets generated from further samples, e.g. a third, and/or fourth, and/or fifth sample, can additionally be merged into the droplets on the chip.

Merging may be carried out by a variety of means. For example, merging may be assisted by the provision of a merging chamber (40), which retains a first droplet between a series of parallel pillars (41), the pillars allowing the carrier fluid to flow around (42) the pillars, such that a second droplet, and optionally a third droplet, merge into a single droplet (35), which, due to surface tension and the pressure applied to the system, then flows out of the merging chamber. The surface properties of the pillars, tubing and walls of the merging chamber, as well as the spacing of the pillars and size of the merging chamber, are determinative for this process. Other droplet merging mechanisms can also be implemented, such as by channel widening to slow down the progress of large droplets; or other mechanical, electrical or optical droplet trapping approaches.

Importantly, the merging of these droplets is controlled, due to the controlled, synchronised delivery of the droplets from the robotic arms to the channels of the microfluidic chip. The invention allows for, and guarantees that, each droplet in a series of droplets will be merged with the intended droplet from a second series of droplets, generated by a separate robotic arm. The controlled merging achieved by the system of the present invention ensures that no droplet fails to receive its intended merger droplet. In one embodiment, one droplet may be, e.g., a substrate, a second droplet may contain a first sample, e.g., a buffer solution, and a third droplet may contain a second sample, e.g., a reagent. As an example, a first sample well (3) contains a substrate, a second sample well (3) contains a reagent, and a third sample well (3) contains a buffer.

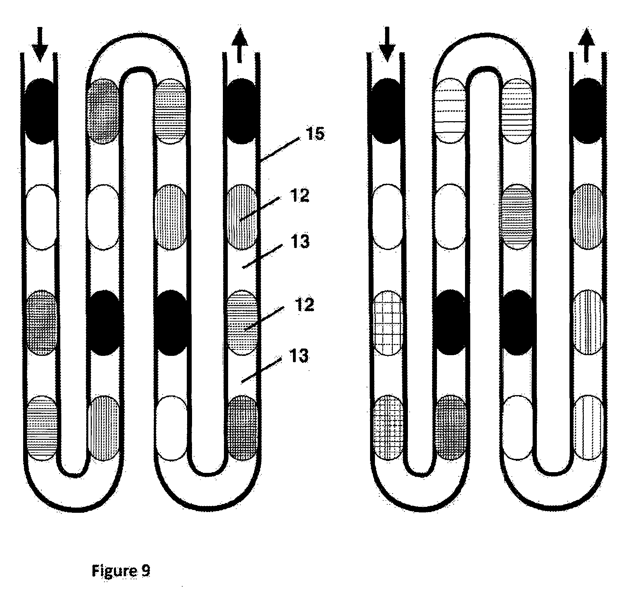

**Figure 9** shows a sequence of different sample droplets (12), separated by carrier fluid (13), in tubing (15) achieved using this invention. The identity of each droplet in a sequence is known.

PRESSURE

To induce droplet flow through the device, negative and/or positive pressure is applied to the system.

Negative pressure is typically applied at the end of the system, or microfluidic chip, distant from the droplet generator, such that the droplets are pulled through the entire system. A suitable negative pressure is in the range of 0-100Pa, or the application of 0.001-0.01 atm of negative pressure (beyond ambient pressure).

Additionally, or alternatively, positive pressure may be applied to the samples to drive the sample droplets through the system. This positive pressure may be the same or different for each of the robotic arms, and is controllable independently in each arm. Positive pressure is typically applied at the end of the system proximate to the droplet generator, such that the droplets are pushed through the system. A suitable positive pressure is in the range of 0-100Pa, or the application of 0.001-0.01 atm of positive pressure (beyond ambient pressure).

In a further embodiment, the system may comprise further points in the system - besides at one or both of the ends of the system distant from or proximate to the droplet generator or synchroniser - where pressure may be applied. In such an embodiment it is essential that the system can be selectively and reversibly sealed between different parts of the apparatus corresponding to different pressure phases. Additional application of positive or negative pressure may assist droplet flow.

The system should be sealed to maintain applied pressure and this can be achieved by means known to the skilled person. Air-tight syringes and air-tight tubing to pump connectors may be used.

Another advantage of the system of the present invention is that no valves are necessary to move sample droplets through the system in either the tubing or the microfluidic chip channels. This leads not only to a much simpler design, but also to a faster operational capability than a system including valves. In contrast, the prior art uses valves to control the flow of droplets. This can lead to contamination between droplets.

MATERIALS

The devices of the system of the present invention are fabricated in a material that has a particular wettability with respect to the continuous phase. That is, the oil phase should wet the channel surfaces and the aqueous droplets should not wet the channel surfaces. Suitable materials include glass, plastics material, and polymers, including polymeric organosilicon compounds.

The devices may, for example, be fabricated from polydimethylsiloxane (PDMS) using known soft lithography methods. The interfacial tension between PDMS and water is approximately 40 mN/m at room temperature. The interfacial tension between PDMS and oil is approximately 1-39 mN/m at room temperature.

The interior wall of the tubing is coated with a material to lend the tubing suitable surface properties. A suitable example for many applications is polytetrafluoroethylene (PTFE; Teflon). Alternatively, the tubing can be made entirely of PTFE or one or more similar materials, such as fluorinated ethylene propylene (FEP), perfluoroalkoxy (PFA). For applications such as single cell studies, the tubing may be coated with a suitable material, such as: gelatine, extra-cellular matrix, collagen, agarose, alginate, poly-L-lysine, phosphatidylcholine, growth factors, glutamate, BSA, Aquapel. Important properties are, for example, hydrophobicity, binding capacity, biological interactions.

The tubing has an inner diameter of between 1$\mu$m to 1 cm, for example, between 20$\mu$m to 1 mm, between 20$\mu$m to 500$\mu$m, between 200$\mu$m to 400$\mu$m for example, approximately 300$\mu$m. The tubing has a length of between 1cm and 1000cm, for example between 10cm and 100cm. This tubing may subsequently be connected to a further device for off-line separation.

The minimum size of droplet for the present invention is such that the droplet completely separates two sequential plugs of carrier fluid, i.e. the droplet must have contact with at least a circumference of the inner diameter of the tube. Minimum droplet size for a particular size of tubing can be determined by the following mathematical formula:

$$V = \frac{4}{3} \cdot \pi \cdot r^3$$

wherein V is the volume of the droplet, and r is the inner radius of the tubing. For the present invention, the internal radius of tubing is preferably in the range of 1$\mu$m to 1cm. For tubing having an inner diameter of 100$\mu$m, the minimum droplet volume is approximately 0.52 nL.

For a droplet that does not have a perfectly spherical shape, the droplet volume approximates to that of a cylinder with hemispherical ends:

$$V = (\pi \cdot r^2 \cdot L) + (4 \cdot \pi / 3 \cdot r^3)$$

wherein L is the length of the cylinder and r is the inner radius of the tubing.

CARRIER FLUID

The carrier fluid may be an oil. Important surface properties of the oil include density, dynamic viscosity, water solubility, thermal conductivity, and boiling point. Examples of suitable oils include, but are not limited to, fluorinated oils, non-fluorinated oils, mineral oils, plant oils, vegetable oils, comestible oils. For example: fluorinated oils such as fc40 or fc328, mineral oil, oleic acid, embryo-tested mineral oil, light mineral oil, heavy mineral oil, PCR mineral oil, AS4 silicone oil, AS 100 silicone oil, AR20 silicone oil, AR 200 silicone oil, AR 1000 silicone oil, AP 100 silicone oil, AP 1000 silicone oil, AP 150 silicone oil, AP 200 silicone oil, CR 200 Silicone oil, DC 200 silicone oil, DC702 silicone oil, DC 710 silicone oil, octanol, decanol, acetophenone, perfluoro-oils perfluorononane, perfluorodecane, perfluorodimethylcylcohexane, perfluoro-1-butanesulfonyl fluoride, perfluoro-1-octanesulfonyl fluoride, perfluoro-1-octanesulfonyl fluoride, nonafluoro-1-butanesulfonyl chloride, nonafluoro-tert-butyl alcohol, perfluorodecanol, perfluorohexane, perfluorooctanol, perfluorodecene, perfluorohexene, perfluorooctene, fuel oil, halocarbon oil 28, halocarbon oil 700, hydrocarbon oil, glycerol, 3M Fluoriner™ fluids (FC-40, FC-43, FC-70, FC-72, FC-77, FC-84. FC-87, FC-3283), soybean oil, castor oil, coconut oil, cedar oil, clove bud oil, fir oil, linseed oil, safflower oil, sunflower oil, almond seed oil, anise oil, clove oil, cottonseed oil, corn oil, croton oil, olive oil, palm oil, peanut oil, bay oil, borage oil, bergamot oil, cod liver oil, macadamia nut oil, camada oil, chamomile oil, citronella oil, eucalyptus oil, fennel oil, lavender oil, lemon oil, nutmeg oil orange oil, petitgrain oil, rose oil, tarragon oil, tung oil, basil oil, birch oil, black pepper oil, birch tar oil, carrot seed oil, cardamom oil, cassia oil, sage oil, cognac oil, copaiba balsam oil, cypress oil, eucalyptus oil, dillweed oil, grape fruit oil, ginger oil, juniper oil, lavender oil, lovage oil, majoram oil, mandarin oil, myrrh oil, neroli oil, olibanum oil, onion oil, paraffin oil, origanum oil, parsley oil, peppermint oil, pimenta leaf oil, sage oil, rosemary oil, rose oil, sandalwood oil, sassafras oil, spearmint oil, thyme oil, transformer oil, verbena oil, and rapeseed oil.

Droplets may be stored as dispersed in the oil phase in the tubing or microfluidic droplet connector, for later use in further analysis or experimental techniques.

ADVANTAGES OF THE SYSTEM OF THE INVENTION

The present invention finds application in high-throughput screening, monitoring and analysing reaction kinetics, diagnostics, drug screening ($k_{cat}$, $k_I$, $k_M$, $IC_{50}$, $EC_{50}$), single cell screening and personalised medicine, such as biomarker screening on small tissue samples, cancer screening, patient profiling *etc.*

There are many advantages associated with the system of the present invention, in terms of sample volume needed, number of assays that can be run per day, and cost. In conventional microfluidic systems, at

least 100 μl of each sample is required, and only 1-10 samples can be tested per day. The repeat rate is high at $10^8$. In High-Throughput Screening (HTS), a single assay requires 100 nL -50 μL of sample. Approximately $10^4$ experiments (i.e. individual data points) can be run per day. However, the cost of HTS is very high, at around $10-100 million for set up and $10,000-$100,000 in maintenance and running costs per year.

In comparison, the system of the present invention has the potential to screen hundreds of samples (e.g. compounds to be tested) per day, and can produce $10^4$ more assays per day than conventional microfluidic systems. Further, the system of the invention can produce $10^4$ samples (e.g. of compounds to be tested) per day, and one sample can be used for $10^8$ assays. With the system of the present invention, 100 assays can be performed per sample (e.g. a drug) per day. Accordingly, thousands of assays can be run per day at a rate of 1 to 100 Hz. The system of the present invention generates approximately 10 droplets per second (10 Hz) per microsampling tip, but can potentially reach much higher frequencies of droplet generation if on-chip splitting of droplet is combined, (e.g. up to 1 kHz).

Another major advantage of the present invention is that only a very small amount of starting sample - about 15-40 μl - is needed. In the sampling system of the present invention, a droplet may typically be around 1 nL in volume. This is sufficient for one repeat of one experimental test. Accordingly, the present sampling system allows for 15.000 - 40.000 droplets to be generated, and 15.000 - 40.000 tests run from one sample. This compares highly favourably to current methods of High Throughput Screening (HTS) or conventional microfluidics. The present invention requires $10^3$ - $10^6$ times less sample volume than HTS.

The sample containers may comprise at least one layer of sample fluid and one layer of carrier fluid. For example, the sample containers may comprise one, two or three layers of sample fluid, in addition to carrier fluid. The layers remain separate on account of different densities of the different fluids. In one embodiment, a layer of carrier fluid may separate two layers of different sample fluids, in a sample container.

The system of the present invention incorporates a very high degree of flexibility in droplet manipulation options. Various illustrative examples are discussed below. This discussion is not intended to be limiting on the scope of the claims.

## 1. Droplet Size

Droplet size and carrier fluid volume can be determined by the residence time of the tip of the microsampling head in the sample solution. The timing is predetermined by programming the electronic means controlling the movement of the microsampling tip and/or of the sample container(s). The minimum droplet size for the present invention is such that the droplet completely separates two sequential plugs of carrier fluid, i.e. the droplet must have contact with at least the inner circumference of the tubing or microchannel.

In any given experiment, droplet size is controllable. For example, all of the droplets may be of substantially the same size, e.g. all droplets generated in a particular experiment will be of substantially the same volume. Alternatively, all droplets of a particular type will be of substantially the same volume, and droplets of different types may have a different volume. Alternatively, all droplets of a particular type may have a different volume.

An example of this is droplets containing an enzyme each being of a first size, and droplets containing a substrate each being of a second size. Droplets of the same type may also be of different sizes, for example, when different amounts of a component are needed, such as a greater amount of a substrate, or different amounts of buffer, in order to create different levels of concentration in a droplet.

By the same means, the volume of the intervening carrier fluid can also be precisely controlled. This precision in droplet size and droplet separation achieved by the sampling capability of the droplet generator allows for precise control on merging two or more droplets together.

## 2. Merging

Merging may be carried out by various methods. Directly in-capillary or in a channel on the microchip, where merging of two or more sequential droplets can be induced by providing a wider section of the channel, a pillar arrangement, or by other methods of droplet trapping. When increasing the internal diameter of the tubing or capillary for an appropriate distance, two or more sequential droplets merge, as the droplets no longer fill an entire cross section of the tube due to hydrophilic attraction. The number of droplets that will merge is dependent on the length of the wider section of the tubing and the separation distance between sequential droplets. The resulting merged droplet will be pulled into the narrower tubing section by the negative pressure applied to the system.

Another merging technique allows droplets to merge in channels on the microchip, where channel junctions exist, as shown in Figure 8. Channels may form a junction at an angle, preferably between about 5° and 175°, more preferably at 45 to 135°, or 80 to 100°, for example at about 90°. One channel may be on the microchip, and the second channel may either be on the microchip, or be tubing, joined to the microchip channel via a connector section.

The present invention allows for precise control of the arrival time of the droplets at merging sites. The present invention ensures that in two separate series of droplets to be merged, each droplet in the first series will arrive at the merging site at the same time as each 'corresponding' droplet from the second series. Due to the precise control over droplet size and droplet spacing achieved by the robotic arms, the present invention allows for precise timing control. This ensures that each droplet from a first series will be merged with a droplet from the second series. Merged droplets will continue to flow through the system.

Where pairs of droplets to be merged contain species that will react with each other, the precise merging also ensures that each droplet pair will be correctly merged as intended, and also allows for the precise determination of the start time of a reaction. This precision is crucial for accurately determining the extent and rate of a reaction.

3. Concentration and Dilution

i) The system of the present invention allows for each of the droplets to be made up to the same concentration or to different concentrations. Droplets of an identical size taken from an identical sample solution will have the same concentration. A smaller droplet will have a relatively lower concentration than a larger droplet taken from the same sample fluid, based purely on volume. Thus, droplet size can determine concentration of individual droplets. The exact concentration of a droplet can also be determined by imaging techniques and droplet size. The concentration of these droplets can be further adjusted by mixing with droplets of a solution, such as a suitable buffer solution.

ii) The concentration of the droplets can be lowered by mixing the droplets from a first sample solution with droplets from a second sample solution. These could, for example, be an analyte for reagent solution and a buffer solution, water, or solvent. The larger the volume of the second droplet added to the first droplet, the more dilute the resulting merged droplet will be.

iii) Droplet size can be adjusted using buffer to ensure that the final size of droplets in a series is the same, if desired.

iv) Dilution series of droplets are directly produced in the device to derive quantitative data. This can be achieved by adjusting the proportions between the amount of reagent and the amount of buffer in a single droplet. In a series of droplets of a particular sample, the concentration can be adjusted to be different in each droplet. For example, the concentration may progressively increase or decrease from the first to the last droplet of the series. Dilution may be carried out on a separate microfluidic chip, located between the microsampling tip and the microfluidic chip on which droplet merging occurs. If dilution occurs in a robotic arm, the device will additionally comprise a reservoir of a solution suitable for dilution of the droplets.

An example of this application is using a reagent at a different and increasing concentration in each droplet to determine a minimum concentration for a reagent to be active. When droplets from a series containing a first reagent (at a constant concentration) are each merged with droplets from a series containing a second reagent (at an increasing concentration), it is possible to determine the minimum concentration needed for the second reagent for a reaction to occur with a first reagent provided at a specific concentration.

v) Other methods are also envisaged as being part of the present invention, such as merging droplets from two series, for example:

(a) a first and second reagent are both increasing in concentration in the series, at the same rate,

(b) a first and second reagent are both increasing in concentration in the series, at a different rate,

(c) a first reagent is decreasing in concentration and a second reagent is at a constant concentration,

(d) a second reagent is decreasing in concentration and a first reagent is at a constant concentration,

(e) a second reagent is at a constant concentration and a first reagent is increasing in concentration.

Various analytical data can be gathered from carrying out such studies. Another advantage of the invention is that thanks to the small sample volumes and lower cost of running the experiments, it is easily possible to obtain vast amounts of very accurate data. Very small changes to the concentration gradient of a substrate can easily be made within a droplet series. This guarantees that the resulting data is extremely accurate and that no data points are missed.

4. Flexibility within one Experimental Run

The system of the present invention incorporates a high degree of flexibility. This is possible due to the highly precise way in which the droplet sampling and synchronised merging can be controlled. The microsampling tip may sample a single droplet from each compound sample to yield a series of different droplets (e.g. Figure 4). Each droplet in the series may be of the same or a different size, in order to result in droplets with an equivalent or different concentration (depending on sample concentrations). Where a sample is of a higher or a lower concentration than other samples, the microsampling tip can aspirate a smaller or larger droplet, in order to obtain a series of different droplets with a constant concentration throughout the series.

If droplets of different sizes are aspirated by the microsampling tip, the on-chip or in-capillary dilution and/or on-chip or in-capillary merging capabilities enable all the droplets in the series to be brought to the same size. This is, for example, achieved by merging different volumes of buffer solution with the sample droplets. The present invention enables precise control, allowing the droplets of the buffer solution aspirated by the microsampling tip to be of the exact volume needed to bring each droplet in the sample series to the same size. The system also allows for no buffer to be added to any sample droplets that do not require any buffer. The device is accurately controllable to ensure that the merging of sample and buffer droplets occurs correctly.

The microsampling tip can also aspirate a plurality of droplets from each sample well. An example use of this would be in conjunction with a dilution study, as described above. For example, a number of droplets of sample A can be aspirated. These may be of an identical size, and hence of an identical concentration. A varying volume of buffer solution can then be added to each droplet (e.g. increasing or decreasing along the series) in order to adjust the size (and relative concentration) of each droplet. Alternatively, the sample droplet concentration can be increasing or decreasing along a series, by aspirating droplets of increasing or decreasing size. An appropriate volume of buffer solution can be added to each droplet in order to adjust the droplet size. Figure 9 shows droplets in a dilution series.

Droplets may be merged with a droplet of a reagent that will react with the species in the sample droplet. This can be carried out in various ways. For example, a series of droplets each containing a different compound from a chemical library can be merged with a droplet containing a specific reagent.

An example of such an application would be in a chemical reaction, such as an enzyme reaction.

A series of identical sample droplets, of an identical concentration, can be merged with a series of droplets containing a differing amount of reagent, for example, the droplets may contain an increasing or decreasing reagent concentration. This enables the lowest feasible reagent concentration to be determined, and also enables the determination of the effect of concentration on reaction rate.

Similarly, a series of identical sample droplets of different concentrations may be merged with a series of reagent droplets each having an identical concentration.

In another embodiment, a series of identical sample droplets of different concentrations may be merged with a series of reagent droplets containing a differing amount of reagent. Each such experiment produces valuable and accurate data.

5. Labelling

A further advantage of the present invention is that droplets do not need to be labelled, because they remain in the same sequence in the tubing or microchannels. The present invention thereby removes any need for incorporating markers or labels in the samples. This is particularly useful in monitoring reaction rates and biological reactions.

6. Screening and Analysis

Using the system of the present invention, droplets containing different reactants can be merged. Upon merging two droplets, each containing a different reagent, a reaction between the reactants can be initiated. The progress of this reaction can be monitored in real time. For example, if a droplet comprising a first reagent is merged with a droplet comprising a second reagent, a reaction between the first and second reagent will take place. This reaction can be monitored using techniques such as fluorescence imaging, laser detection methods, Raman scattering or label-free detection such as absorbance, changes in refractive index or bioluminescence.

Series of droplets can also be repeatedly run forwards and backwards across a laser or fluorescence beam in order to analyse the time course of the reaction. Data from each individual droplet can then be gathered.

It is also possible to selectively consider only droplets of interest. For example, it is possible to study only droplets containing a first and second reagent at specific concentration levels of interest. This study gives data on the relationship of the reaction rate with the concentrations of each reagent.

7. Continuous Screening (on-chip or in capillary)

Sequences of droplets as micro-reactors are analysed for a readout (fluorescence, absorbance). The readout measurement can be taken continuously over a time-course or at specific time points either in continuous-flow, or statically with droplet trapping or storage. Detection of the reaction can be done either on chip or in capillary. Because the droplets are analysed in sequence, multiple readouts can be assigned to each droplet giving real-time data.

8. Cell Screening

In single cell screening, such as biopsies, or tissue samples, and screening clinical trial candidates, one cell is contained in one droplet. This allows for the screening and monitoring of a single cell. The data obtained from these single cell experiments is highly specific and therefore extremely valuable. The isolation of single cells into droplet compartments yields more accurate statistical data. Current experimental techniques allow for averaging of data over 3000 cells. The system of the present invention allows for the screening of much larger numbers of cells (up to $10^6$ to $10^8$ cells). In general, detection of cell assays (e.g. signalling, surface binding) through fluorescence or absorbance measurements can be used for toxicity prediction, stem cell studies, diagnostic applications (e.g. Cancer screening and personalised medicine, for diseases, including, but not limited to Alzheimer's Disease, Malaria).

### 9. Development of Assays For Screening

The present invention allows for the development of assays for screening a multitude of samples or analytes across a variety of applications and disciplines. These encompass:

- Drug Detection (e.g. Performance-enhancing drugs)
- Drug Discovery (e.g. Inhibitor screening, toxicity assays, high-throughput screening)
- Diagnostics (biomarkers)
- Forensics
- Veterinarian Diseases
- Food, Agricultural, Environmental testing
- Biochemical warfare detection

### 10. Analysis

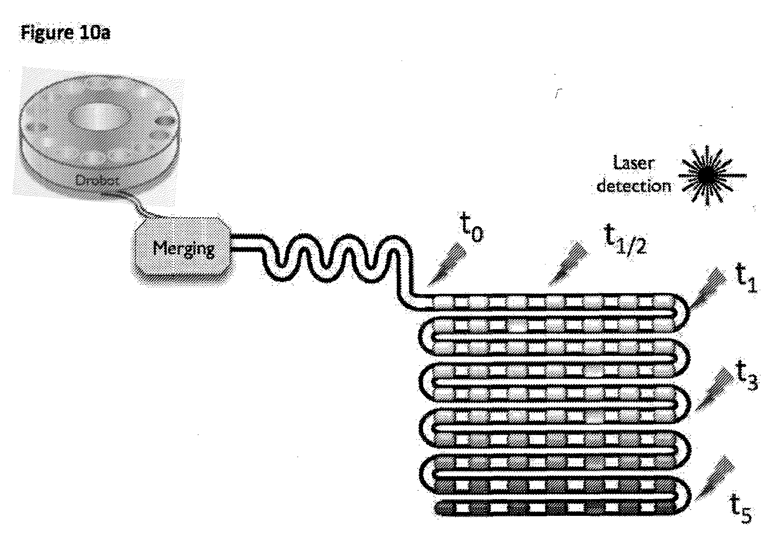

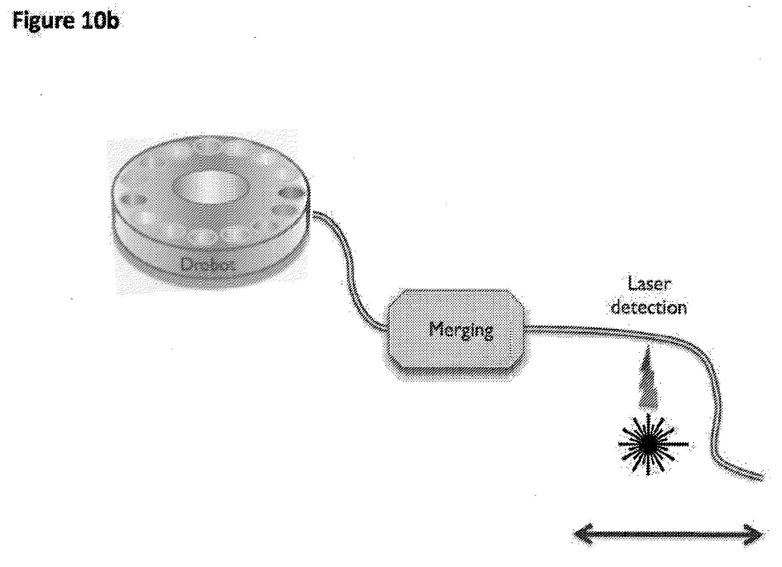

Analysis of droplets can be carried out by various methods. Appropriate detection techniques include high-speed imaging, Laser detection, absorbance and luminescence (fluorescence, phosphorescence, chemi- and bio-luminescence). Figures 10a and 10b show a set up for ultra fast kinetic analysis using laser detection. Merged droplets flow through the capillary or leave the microfluidic chip via tubing or another capillary, retaining the same order, and separated by carrier fluid. The tubing may be arranged in one straight line, as a coil, or in a series of loops, depending on analytical and spatial constraints. Laser detection may be carried out at several locations, in order to carry out analysis over a time period. Droplet velocity may be determined by recording a video of the droplets. In this way, the time point of each reading (laser detection) can be determined, and the extent of a reaction can be followed over a time course. Known standard droplets can be analysed in order to correct for variations in focus.

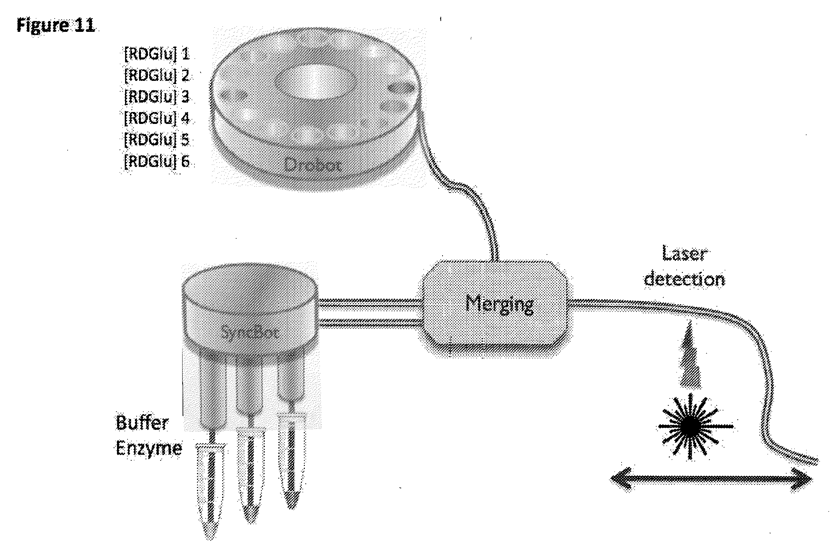

It is also possible to control the droplets to be moved forwards and backwards over a point of detection, such as a laser, in order to obtain data for each droplet over a time course. This can be achieved by adjusting the pressure in the system. This is illustrated in Figures 11-14.

EXAMPLES

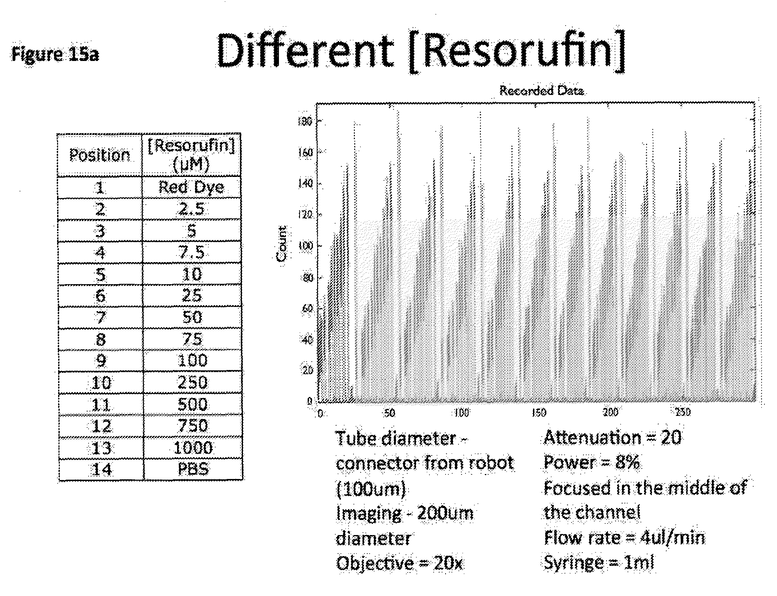

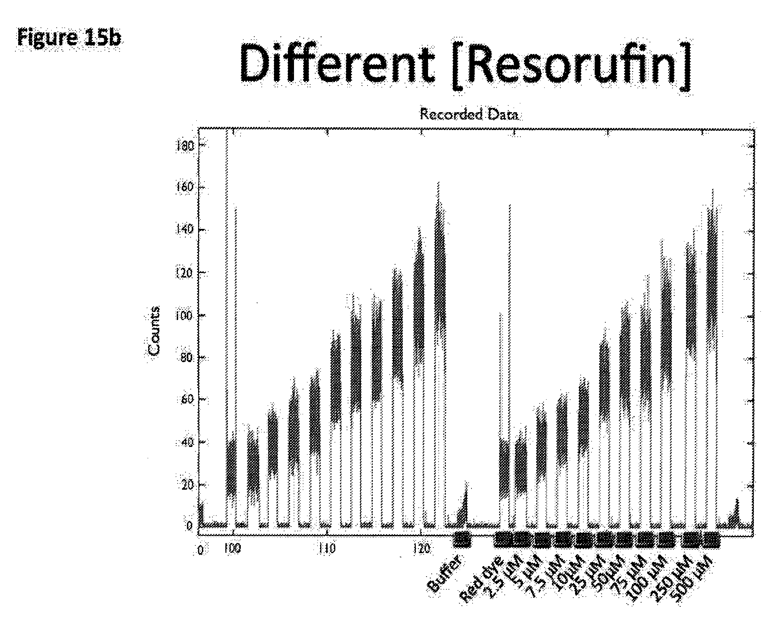

Further data for the examples described below are shown in Figures 15-19.

### Example 1

This example relates to the reaction of enzyme β-glucosidase and different concentrations of substrate RDGlu (Resorufin-D-Glucopyranoside) in buffer PBS (100mM, pH 7.4). The reaction product is resorufin, a fluorescent dye. The system configuration is shown in Figure 11. Monitoring at $\lambda_{ex}$ 571 nm; $\lambda_{em}$ 590 nm.

As a first step, β-glucosidase is applied to a cover-slip, with the lowest and highest RDGlu concentrations to be used. This enables the determination of the fastest and slowest time taken to reach saturation. Then, samples of β-glucosidase, and samples with different concentrations of RDGlu are made up immediately prior to loading the samples into the droplet generator. Droplet sequences of RDGlu with different concentrations are generated. The droplet synchroniser generates droplets of the enzyme and buffer. On a merging chip, 10 to 50 repeats of each reaction of a different RDGlu concentration and β-glucosidase and buffer are merged. Droplets flow off the microfluidic chip into a section of tubing, where analysis and reaction monitoring is carried out by laser detection methods. Droplets reach the laser approximately 30-120 seconds after droplet generation. Droplets are run back and forth over the laser to yield time course data.

### Example 2

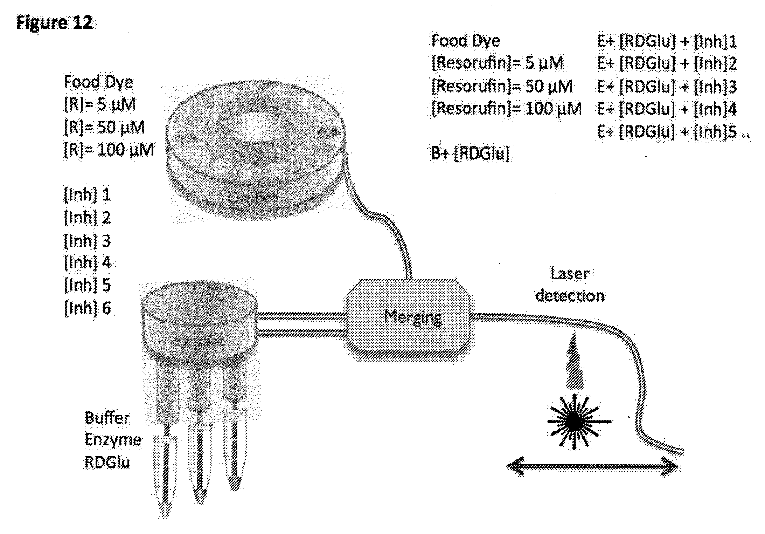

The reaction of Example 1 is repeated, this time generating droplets of different concentrations of an inhibitor and food dye in the droplet generator, and generating droplets of buffer, enzyme, and different concentrations of RDGlu in the droplet synchroniser. On a merging chip, 10 to 50 repeats of each reaction are merged (Figure 12).

### Example 3

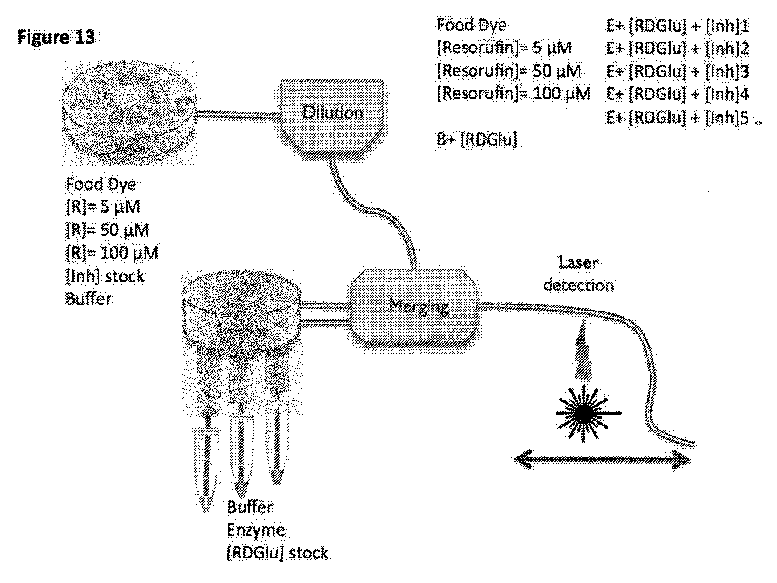

The reaction of Example 2 is repeated, this time generating droplets of an inhibitor from a stock solution, using buffer to dilute the concentration, and food dye in the droplet generator, and generating droplets of buffer, enzyme, and RDGlu from a stock solution in the droplet synchroniser. On a merging chip, 10 to 50 repeats of each reaction are merged (Figure 13).

### Example 4

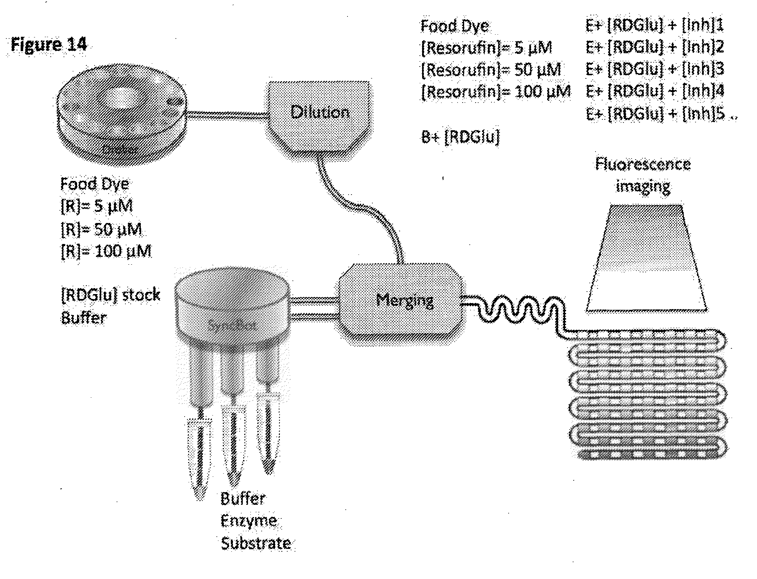

The reaction of Example 1 is repeated, this time generating droplets of RDGlu from a stock solution, buffer and food dye in the droplet generator, and generating droplets of buffer, enzyme, and substrate in the droplet synchroniser. Fluorescence imaging is used to determine the extent and rate of the reaction in the droplets. On a merging chip, 10 to 50 repeats of each reaction are merged (Figure 14).

Figure 15 shows Reproducibility: 12 repeats of a given sequence (12 samples, described in the table and shown above) and details of 2 repeats (left).

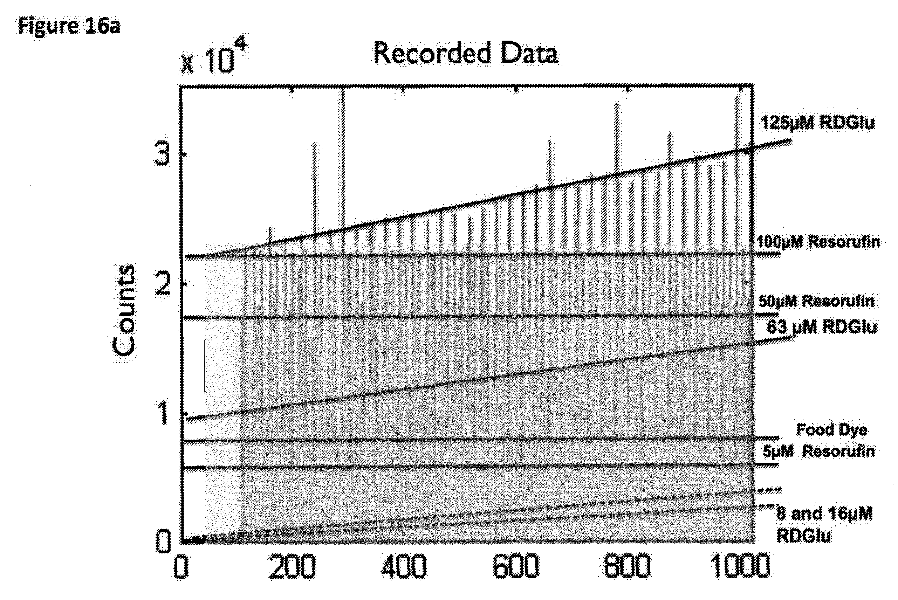

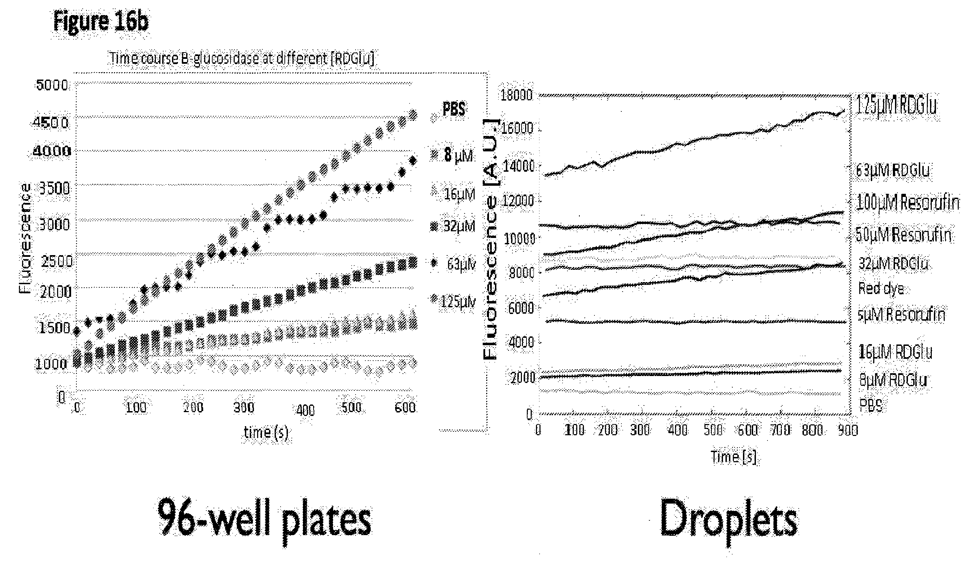

Figure 16 shows kinetic data obtained in droplets for ß-glucosidase (a diabetes Type I target)

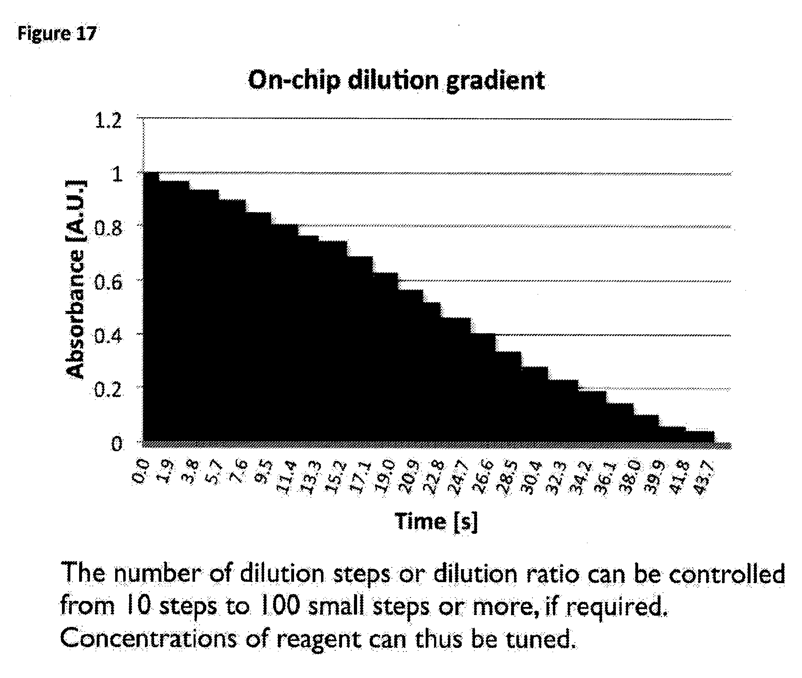

Figure 17 shows an example of dilution steps created with the invention. The number of dilution steps or dilution ratio can be controlled from 10 steps to 100 small steps or more, if required. Concentrations of reagent can thus be tuned.

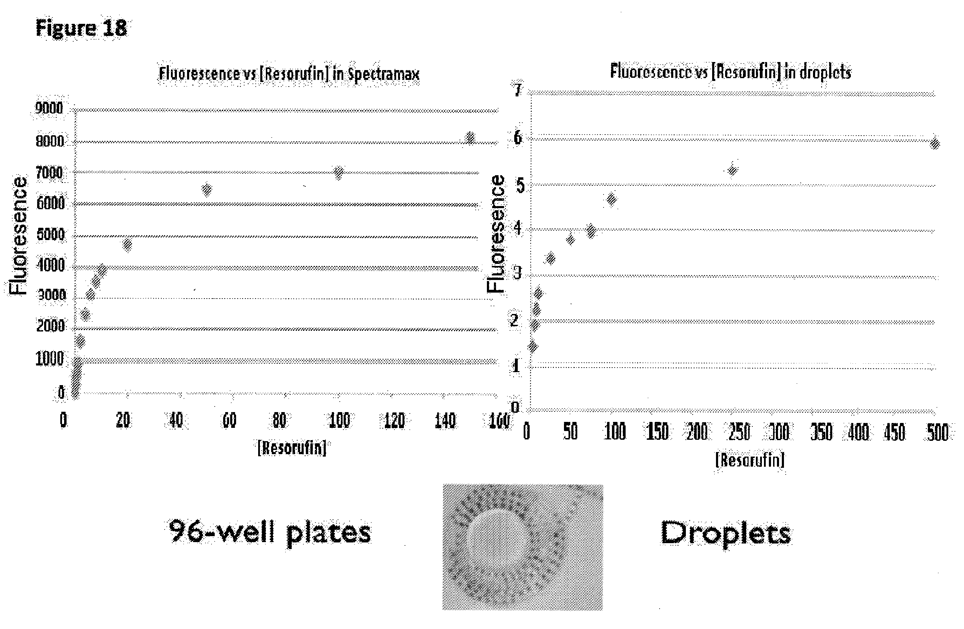

Figure 18 show a comparison of fluorescence titration (Resorufin) in a plate reader (96-well plate) and in droplets. Droplets-on-demand are supplied by a robotic picolitre compound dispenser that can supply different contents in a known sequence into droplets so that the identity of droplet contents can be decoded. The readout for a fluorescence titration shows identical trends to one measured in a microtiter plate.

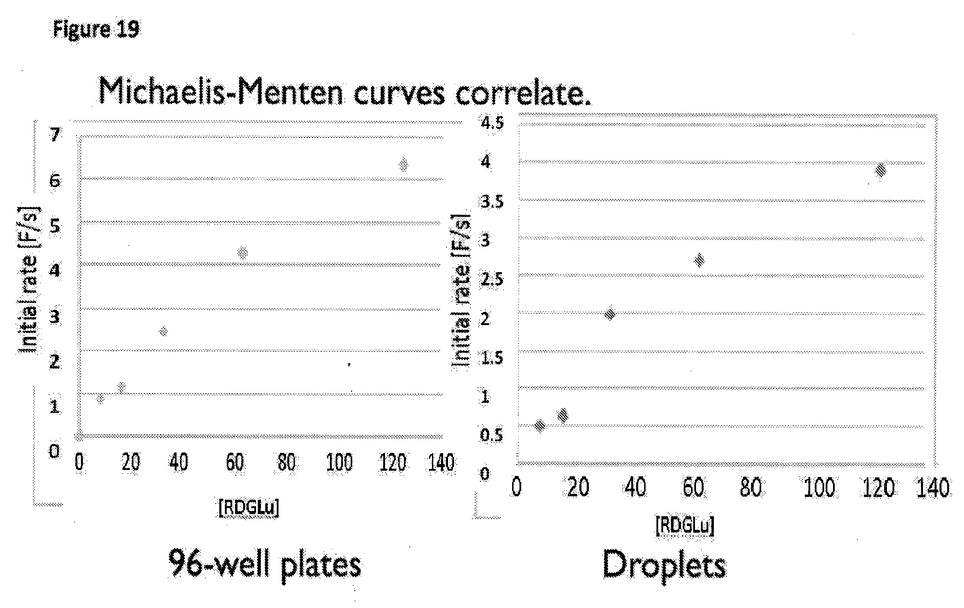

Figure 19 shows a comparison of Michaelis-Menten kinetic curves obtained in a plate-reader and in droplets. Both give identical results.


**Claims**

1. A microfluidic system for the controlled generation of droplets of a sample fluid, separated by a carrier fluid that is immiscible with the sample fluid, the system comprising:

   o a reservoir for containing a body of sample fluid and a body of carrier fluid in distinct layers, separated by an interface;
   o a droplet generator, comprising an opening for the controlled admission of the sample fluid and the carrier fluid into the generator from the reservoir;
   o a translator for moving the droplet generator opening relative to reservoir, or for moving the reservoir relative to the droplet generator opening; and
   o an active pressure system for causing droplets of the sample fluid or the carrier fluid to be drawn into the opening;

   wherein the active pressure system comprises at least one source of pressure for moving the droplets through the system;
   whereby the opening can be moved relative to reservoir, or the reservoir can be moved relative to the opening, for the opening to be alternatively located in the sample or in the carrier fluid by moving across the interface between the sample and the carrier fluid and draw successive droplets of the sample and the carrier fluid into the droplet generator; **characterised in that** the droplet generator opening draws a sample droplet from below the sample reservoir.

2. The microfluidic system as claimed in claim 1, wherein the droplet generator opening is an open end of a capillary or of tubing.

3. The microfluidic system as claimed in claim 1 or claim 2, wherein the system additionally comprises a microfluidic chip supporting at least one channel or capillary to conduct one or more droplets in a carrier fluid, wherein the system additionally comprises a conduit, for example a section of tubing or capillary, connecting the opening with the microfluidic chip.

4. The microfluidic system as claimed in any one of claims 1 to 3, wherein the opening and the reservoir are moveable relative to each other, to selectively locate the opening in the sample fluid and the carrier fluid to thereby form droplets of sample fluid, interspersed by droplets of carrier fluid.

5. The microfluidic system as claimed in any one of claims 1 to 4, wherein the reservoir contains at least one sample fluid, and a carrier fluid, in distinct layers.

6. The microfluidic system of claim 5, wherein the reservoir is in the form of a microtiter plate.

7. The microfluidic system as claimed in any one of claims 1 to 6, wherein the system comprises two or more droplet generator openings, and the movement of each opening is independently controllable to draw droplets of sample fluid that flow from the openings through the respective conduit to a microfluidic chip, or a tubing or a capillary, where two or more droplets can be merged into one droplet either directly in the capillary or on a microfluidic chip.

8. The microfluidic system as claimed in claim 7, wherein the droplets that are merged originate from the same opening or from different openings.

9. The microfluidic system of any of claim 1-8, wherein the system is integrated with a further instrument, for example, a mass spectrometer.

10. The microfluidic system as claimed in any one of claims 1 to 9, wherein two or more sample fluid droplets that are separated by a carrier fluid that is immiscible with the sample fluid, are merged into a single mixed sample fluid droplet in a controlled manner either in sequence in a capillary or tubing, at a predetermined point in time, or on a microfluidic chip at a predetermined location and point in time.

11. The microfluidic system as claimed in claim 10, wherein the two or more droplets are generated respectively by two or more droplet generators that are set up in parallel and wherein the two or more droplet generators are synchronised to each generate droplets

    a) at substantival the same time; and/or
    b) to arrive at a predetermined location at substantially the same time;

to generate synchronised droplets and enable merging of the synchronised droplets.

12. The microfluidic system as claimed in any one of claims 1-11, wherein each sample fluid is independently selected from the group comprising:

    a compound from a chemical library, a substrate, a reagent, an enzyme, a buffer, a tissue sample, a human biological sample extract,
    a bodily fluid, a cell, a cell component, a nucleic acid, a sequence of DNA, and / or an entrapment bead.

13. The microfluidic system as claimed in any one of claims 1-12, wherein a droplet or a merged droplet of sample fluid is subsequently used in an assay system; for example wherein the assay is for binding kinetics, cancer screening, diagnostics, patient profiling, or high throughput screening.

14. A method of analysing a droplet, or a merged droplet resulting from two or more droplets, or a sequence of merged droplets, generated by the microfluidic system as claimed in any one of claims 1 to 13, wherein the analysis is carried out on the droplet in the tubing or in a capillary or on a microchip.

15. The method of claim 14, wherein an analytical, imaging, or diagnostic instrument is integrated into, or provided as a separate module from, but connected to, the microfluidic system as claimed in any one of claims 1 to 13; for example wherein the analytical or diagnostic instrument is selected from an instrument for carrying out capillary electrophoresis, mass spectrometry, absorbance detection, fluorescence detection, luminescence detection, bioluminescence detection, or phosphorescence detection, or a quality control instrument, for example, for testing the quality of air, petroleum, gas, or a chemical compound.

**Patentansprüche**

1. Mikrofluidisches System zur kontrollierten Erzeugung von Tröpfchen aus einem Probenfluid, die durch ein Trägerfluid getrennt sind, welches mit dem Probenfluid nicht mischbar ist, wobei das System aufweist:

    o ein Reservoir für das Enthalten eines Körpers des Probenfluids und eines Körpers des Trägerfluids in verschiedenen Schichten, die durch eine Grenzfläche getrennt sind;
    o einen Tröpfchengenerator, der eine Öffnung für die kontrollierte Aufnahme des Probenfluids und des Trägerfluids aus dem Reservoir in den Generator aufweist;
    o eine Verschiebeeinrichtung zum Bewegen der Tröpfchengeneratoröffnung relativ zu dem Reservoir oder zum Bewegen des Reservoirs relativ zu der Tröpfchengeneratoröffnung; und
    o ein aktives Drucksystem zum Bewirken, dass Tröpfchen des Probenfluids oder des Trägerfluids in die Öffnung gezogen werden;

wobei das aktive Drucksystem mindestens eine Druckquelle aufweist, um die Tröpfchen durch das System zu bewegen, wobei die Öffnung relativ zu dem Reservoir bewegt werden kann oder das Reservoir relativ zu der Öffnung bewegt werden kann, damit die Öffnung durch Bewegen über die Grenzfläche zwischen dem Proben- und dem Trägerfluid wahlweise in dem Proben- oder in dem Trägerfluid angeordnet ist und aufeinanderfolgend Tröpfchen des Proben- und des Trägerfluids in den Tröpfchengenerator zieht; **dadurch gekennzeichnet, dass** die Tropfengeneratoröffnung ein Probentröpfchen von unterhalb des Probenreservoirs zieht.

2. Mikrofluidisches System nach Anspruch 1, wobei die Tröpfchengeneratoröffnung ein offenes Ende einer Kapillare oder eines Schlauches ist.

3. Mikrofluidisches System nach Anspruch 1 oder Anspruch 2, wobei das System zusätzlich einen mikrofluidischen Chip umfasst, der mindestens einen Kanal oder eine Kapillare trägt, um ein oder mehrere Tröpfchen in einem Trägerfluid zu leiten, wobei das System zusätzlich eine Leitung aufweist, beispielsweise einen Schlauchabschnitt oder eine Kapillare, welche die Öffnung mit dem mikrofluidischen Chip verbindet.

4. Mikrofluidisches System nach einem der Ansprüche 1 bis 3, wobei die Öffnung und das Reservoir relativ zueinander beweglich sind, um selektiv die Öffnung in dem Probenfluid und dem Trägerfluid anzuordnen, um dadurch Tröpfchen des Probenfluids zu bilden, die von dazwischenliegenden Tröpfchen des Trägerfluids unterbrochen sind.

5. Mikrofluidisches System nach einem der Ansprüche 1 bis 4, wobei das Reservoir mindestens ein Probenfluid und ein Trägerfluid in verschiedenen Schichten enthält.

6.  Mikrofluidisches System nach Anspruch 5, wobei das Reservoir in der Form einer Mikrotiterplatte ist.

7.  Mikrofluidisches System nach einem der Ansprüche 1 bis 6, wobei das System zwei oder mehrere Tröpfchengeneratoröffnungen aufweist und die Bewegung von jeder Öffnung unabhängig steuerbar ist, um Tröpfchen des Probenfluids zu ziehen, die von den Öffnungen durch die entsprechende Leitung zu einem mikrofluidischen Chip oder einem Schlauch oder einer Kapillare fließen, wobei zwei oder mehr Tröpfchen entweder direkt in der Kapillare oder auf einem mikrofluidischen Chip zu einem Tröpfchen zusammengeführt werden können.

8.  Mikrofluidisches System nach Anspruch 7, wobei die Tröpfchen, die zusammengeführt werden, aus der gleichen Öffnung oder aus verschiedenen Öffnungen stammen.

9.  Mikrofluidisches System nach einem der Ansprüche 1 - 8, wobei das System in einem weiteren Gerät, beispielsweise einem Massenspektrometer, integriert ist.

10. Mikrofluidisches System nach einem der Ansprüche 1 bis 9, wobei zwei oder mehr Probenfluidtröpfchen, die durch ein Trägerfluid getrennt sind, das mit dem Probenfluid nicht mischbar ist, zu einem einzigen gemischten Probenfluidtröpfchen in kontrollierter Weise entweder der Reihe nach in einer Kapillare oder einem Schlauch, an einem vorbestimmten Zeitpunkt oder auf einem mikrofluidischen Chip an einem vorbestimmten Ort und Zeitpunkt zusammengeführt werden.

11. Mikrofluidisches System nach Anspruch 10, wobei die zwei oder mehr Tröpfchen entsprechend durch zwei oder mehr Tröpfchengeneratoren erzeugt werden, die parallel eingerichtet sind und wobei die zwei oder mehreren Tröpfchengeneratoren synchronisiert sind, um jeweils Tröpfchen zu erzeugen

> a) im Wesentlichen zur gleichen Zeit; und/oder
> b) um an einer vorbestimmten Stelle im Wesentlichen zur gleichen Zeit anzukommen;

um synchronisierte Tröpfchen zu erzeugen und um das Zusammenführen der synchronisierten Tröpfchen zu ermöglichen.

12. Mikrofluidisches System nach einem der Ansprüche 1 - 11, wobei jedes Probenfluid unabhängig ausgewählt ist aus der Gruppe, bestehend aus: einer Verbindung aus einer chemischen Bibliothek, einem Substrat, einem Reagens, einem Enzym, einem Puffer, einer Gewebeprobe, einem menschlichen biologischen Probenextrakt, einem Körperfluid, einer Zelle, einer Zellkomponente, einer Nukleinsäure, einer DNA-Sequenz und/oder einem Einschluss-Kügelchen.

13. Mikrofluidisches System nach einem der Ansprüche 1 - 12, wobei ein Tröpfchen oder ein zusammengeführtes Tröpfchen Probenfluid anschließend in einem Assaysystem verwendet wird; wobei zum Beispiel der Assay für Bindungskinetiken, Krebs-Screening, Diagnostik, Erstellung von Patientenprofilen oder High-Throughput-Screening ist.

14. Verfahren zum Analysieren eines Tröpfchens oder eines zusammengeführten Tröpfchens, das aus zwei oder mehr Tröpfchen hervorgeht, oder einer Sequenz von zusammengeführten Tröpfchen, die durch das mikrofluidische System erzeugt wird, wie nach einem der Ansprüche 1 bis 13 beansprucht, wobei die Analyse an dem Tröpfchen in dem Schlauch oder in einer Kapillare oder auf einem Mikrochip durchgeführt wird.

15. Verfahren nach Anspruch 14, wobei ein analytisches, bildgebendes oder diagnostisches Instrument integriert ist in das mikrofluidische System, wie es in einem der Ansprüche 1 bis 13 beansprucht ist, oder als ein separates Modul davon, jedoch damit verbunden, vorgesehen ist; wobei zum Beispiel das analytische oder diagnostische Instrument ausgewählt ist aus einem Instrument zur Durchführung von Kapillarelektrophorese, Massenspektrometrie, Absorptions-Detektion, Fluoreszenz-Detektion, Lumineszenz-Detektion, Biolumineszenz-Detektion oder Phosphoreszenz-Detektion, oder einem Instrument zur Qualitätskontrolle, beispielsweise für die Prüfung der Qualität von Luft, Erdöl, Gas oder einer chemischen Verbindung.

**Revendications**

1.  Système microfluidique pour la production maîtrisée de gouttelettes d'un fluide échantillon, séparé par un fluide porteur qui est non miscible avec le fluide échantillon, le système comprenant :

> - un réservoir pour contenir une masse de fluide échantillon et une masse de fluide porteur dans des couches distinctes, séparées par une interface ;
> - un générateur de gouttelettes, comprenant une ouverture pour l'admission maîtrisée du fluide échantillon et du fluide porteur dans le générateur à partir du réservoir ;
> - un dispositif de translation pour déplacer l'ouverture de générateur de gouttelettes par rapport au réservoir, ou pour déplacer le réservoir par rapport à l'ouverture de générateur de gouttelettes ; et

- un système de pression active pour amener les gouttelettes du fluide échantillon ou du fluide porteur à être entraînées dans l'ouverture ;

dans lequel le système de pression active comprend au moins une source de pression pour déplacer les gouttelettes à travers le système ;

de sorte que l'ouverture peut être déplacée par rapport au réservoir, ou le réservoir peut être déplacé par rapport à l'ouverture, pour que l'ouverture soit placée en alternance dans l'échantillon ou dans le fluide porteur en se déplaçant d'un bout à l'autre de l'interface entre l'échantillon et le fluide porteur et absorbe des gouttelettes successives de l'échantillon et du fluide porteur dans le générateur de gouttelettes ; **caractérisé en ce que** l'ouverture de générateur de gouttelettes absorbe une gouttelette d'échantillon à partir du dessous du réservoir d'échantillon.

2. Système microfluidique selon la revendication 1, dans lequel l'ouverture de générateur de gouttelettes est une extrémité ouverte d'un capillaire ou d'un tubage.

3. Système microfluidique selon la revendication 1 ou la revendication 2, dans lequel le système comprend de plus une puce microfluidique supportant au moins un canal ou capillaire pour conduire une ou plusieurs gouttelettes dans un fluide porteur, dans lequel le système comprend de plus un conduit, par exemple une section de tubage ou de capillaire, reliant l'ouverture à la puce microfluidique.

4. Système microfluidique selon l'une quelconque des revendications 1 à 3, dans lequel l'ouverture et le réservoir sont mobiles l'un par rapport à l'autre, pour placer de manière sélective l'ouverture dans le fluide échantillon et le fluide porteur pour former de ce fait des gouttelettes de fluide échantillon, intercalées avec des gouttelettes de fluide porteur.

5. Système microfluidique selon l'une quelconque des revendications 1 à 4, dans lequel le réservoir contient au moins un fluide échantillon et un fluide porteur, dans des couches distinctes.

6. Système microfluidique selon la revendication 5, dans lequel le réservoir est sous la forme d'une plaque de microtitres.

7. Système microfluidique selon l'une quelconque des revendications 1 à 6, dans lequel le système comprend deux ou plusieurs ouvertures de générateur de gouttelettes, et le mouvement de chaque ouverture peut être maîtrisé indépendamment pour absorber les gouttelettes de fluide échantillon qui s'écoulent en provenance des ouvertures à travers le conduit respectif jusqu'à une puce microfluidique, ou un tubage ou un capillaire, où deux ou plusieurs gouttelettes peuvent être fusionnées en une seule gouttelette soit directement dans le capillaire soit sur une puce microfluidique.

8. Système microfluidique selon la revendication 7, dans lequel les gouttelettes qui sont fusionnées proviennent de la même ouverture ou d'ouvertures différentes.

9. Système microfluidique selon l'une quelconque des revendications 1 à 8, dans lequel le système est intégré avec un instrument supplémentaire, par exemple, un spectromètre de masse.

10. Système microfluidique selon l'une quelconque des revendications 1 à 9, dans lequel deux ou plusieurs gouttelettes de fluide échantillon qui sont séparées par un fluide porteur qui est non miscible dans le fluide échantillon, sont fusionnées en une seule gouttelette de fluide échantillon mixte d'une façon maîtrisée soit en séquence dans un capillaire ou un tubage, à un point prédéterminé dans le temps, soit sur une puce microfluidique à un emplacement et à un point dans le temps prédéterminés.

11. Système microfluidique selon la revendication 10, dans lequel les deux ou plusieurs gouttelettes sont respectivement produites par deux ou plusieurs générateurs de gouttelettes qui sont mis en parallèle et dans lequel les deux ou plusieurs générateurs de gouttelettes sont synchronisés pour produire chacun des gouttelettes

a) sensiblement en même temps ; et/ou
b) pour parvenir à un emplacement prédéterminé sensiblement en même temps ;

pour produire des gouttelettes synchronisées et permettre la fusion des gouttelettes synchronisées.

12. Système microfluidique selon l'une quelconque des revendications 1 à 11, dans lequel chaque fluide échantillon est indépendamment sélectionné à partir du groupe comprenant :

un composé provenant d'une banque chimique, un substrat, un réactif, une enzyme, un tampon, un échantillon de tissu, un extrait d'échantillon biologique humain, un fluide corporel, une cellule, un composant cellulaire, un acide nucléique, une séquence d'ADN et/ou une perle d'encapsulation.

**13.** Système microfluidique selon l'une quelconque des revendications 1 à 12, dans lequel une gouttelette ou une gouttelette fusionnée de fluide échantillon est utilisée par la suite dans un système de dosage ; par exemple dans lequel le dosage sert à la cinétique de liaison, le dépistage du cancer, des diagnostics, le profilage du patient, ou le criblage à haut débit.

**14.** Procédé d'analyse d'une gouttelette, ou d'une gouttelette fusionnée résultant de deux ou plusieurs gouttelettes, ou d'une séquence de gouttelettes fusionnées, produites par le système microfluidique selon l'une quelconque des revendications 1 à 13, dans lequel l'analyse est effectué sur la gouttelette dans le tubage ou dans un capillaire ou sur une micropuce.

**15.** Procédé selon la revendication 14, dans lequel un instrument analytique, d'imagerie, ou de diagnostic est intégré dans le, ou fourni en tant que module distinct du, mais relié au, système microfluidique selon l'une quelconque des revendications 1 à 13 ; par exemple dans lequel l'instrument analytique ou de diagnostic est sélectionné à partir d'un instrument pour effectuer une électrophorèse capillaire, une spectrométrie de masse, une détection d'absorbance, une détection de fluorescence, une détection de luminescence, une détection de bioluminescence, ou une détection de phosphorescence, ou un instrument de contrôle de qualité, par exemple, pour évaluer la qualité de l'air, de pétrole, de gaz, ou d'un composé chimique.

**Figure 1B**

**Figure 1C**

**Figure 2**

Figure 3

Figure 4

Figure 5

Figure 6

Figure 7

Figure 8

Figure 9

Figure 10a

Figure 10b

EP 2 864 048 B1

Figure 11

**Figure 12**

Food Dye
[R]= 5 µM
[R]= 50 µM
[R]= 100 µM

[Inh] 1
[Inh] 2
[Inh] 3
[Inh] 4
[Inh] 5
[Inh] 6

Food Dye
[Resorufin]= 5 µM
[Resorufin]= 50 µM
[Resorufin]= 100 µM

B+ [RDGlu]

E+ [RDGlu] + [Inh]1
E+ [RDGlu] + [Inh]2
E+ [RDGlu] + [Inh]3
E+ [RDGlu] + [Inh]4
E+ [RDGlu] + [Inh]5 ..

Buffer
Enzyme
RDGlu

Laser detection

Merging

EP 2 864 048 B1

**Figure 13**

Food Dye
[R]= 5 µM
[R]= 50 µM
[R]= 100 µM
[Inh] stock
Buffer

Buffer
Enzyme
[RDGlu] stock

Food Dye
[Resorufin]= 5 µM
[Resorufin]= 50 µM
[Resorufin]= 100 µM

B+ [RDGlu]

E+ [RDGlu] + [Inh]1
E+ [RDGlu] + [Inh]2
E+ [RDGlu] + [Inh]3
E+ [RDGlu] + [Inh]4
E+ [RDGlu] + [Inh]5 ..

Dilution

Merging

Laser detection

**Figure 14**

Food Dye
[R]= 5 μM
[R]= 50 μM
[R]= 100 μM

[RDGlu] stock
Buffer

Buffer
Enzyme
Substrate

Dilution

Merging

Food Dye
[Resorufin]= 5 μM
[Resorufin]= 50 μM
[Resorufin]= 100 μM

B+ [RDGlu]

E+ [RDGlu] + [Inh]1
E+ [RDGlu] + [Inh]2
E+ [RDGlu] + [Inh]3
E+ [RDGlu] + [Inh]4
E+ [RDGlu] + [Inh]5 ..

Fluorescence
imaging

Figure 15a

# Different [Resorufin]

Recorded Data

| Position | [Resorufin] (µM) |
|----------|------------------|
| 1 | Red Dye |
| 2 | 2.5 |
| 3 | 5 |
| 4 | 7.5 |
| 5 | 10 |
| 6 | 25 |
| 7 | 50 |
| 8 | 75 |
| 9 | 100 |
| 10 | 250 |
| 11 | 500 |
| 12 | 750 |
| 13 | 1000 |
| 14 | PBS |

Tube diameter - connector from robot (100um)
Imaging - 200um diameter
Objective = 20x

Attenuation = 20
Power = 8%
Focused in the middle of the channel
Flow rate = 4ul/min
Syringe = 1ml

Figure 15b

EP 2 864 048 B1

Figure 16a

Figure 16b

96-well plates

Droplets

Figure 17

## On-chip dilution gradient

The number of dilution steps or dilution ratio can be controlled from 10 steps to 100 small steps or more, if required. Concentrations of reagent can thus be tuned.

Figure 18

Fluorescence vs [Resorufin] in Spectramax

Fluorescence vs [Resorufin] in droplets

96-well plates

Droplets

EP 2 864 048 B1

Figure 19

# Michaelis-Menten curves correlate.

96-well plates

Droplets

Figure 20

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20090288710 A
- US 7129091 B
- US 20060094119 A

### Non-patent literature cited in the description

- *Current Opinion in Chemical Biology,* 2010, vol. 14, 1-8
- *Mol. BioSyst,* 2009, vol. 5, 1392-1404
- *Lab Chip,* 2008, vol. 8, 1244-1254
- **NIU et al.** *Nature Chemistry,* 2011, vol. 3, 437-442
- **WHITESIDES, G.M. ; OSTUNI, E.S. ; TAKAYAMA, S. ; JIANG, X. ; INGBER, D.E.** Soft Lithography in Biology and Biochemistry. *Ann. Rev. Biomed. Eng,* 2001, vol. 3, 335-373